# EUROPEAN PATENT SPECIFICATION

(11) **EP 4 402 271 B1**
(45) Date of publication and mention of the grant of the patent: **10.09.2025**
(21) Application number: 22789883.0
(22) Date of filing: 15.09.2022
(51) Int. Cl.: C12N 15/82, C12N 9/02, C12N 9/10, C12P 7/44, A23L 2/02, A23L 27/00, A61K 36/88

(54) **TRANSGENIC PLANTS PRODUCING HIGH LEVELS OF APOCAROTENOIDS COMPOUNDS AND USES THEREOF**
TRANSGENE PFLANZEN, DIE HOHE MENGEN AN APOCAROTENOIDVERBINDUNGEN PRODUZIEREN, UND VERWENDUNGEN DAVON
PLANTES TRANSGÉNIQUES PRODUISANT DES NIVEAUX ÉLEVÉS DE COMPOSÉS D'APOCAROTÉNOÏDES ET LEURS UTILISATIONS

(30) Priority: 15.09.2021 EP 21382833
(43) Date of publication of application: 24.07.2024
(73) Proprietor: Universidad de Castilla-la Mancha, 13071 Ciudad Real (ES); Consejo Superior De Investigaciones Científicas, 28006 Madrid (ES); Universitat Politècnica de València, 46002 Valencia (ES)
(72) Inventor: GÓMEZ GÓMEZ, María Lourdes, 13071 Ciudad Real (ES); AHRAZEM EL KADIRI, Oussama, 13071 Ciudad Real (ES); GRANELL RICHART, Antonio, 13071 Ciudad Real (ES); RUBIO MORAGA, Ángela, 13071 Ciudad Real (ES); ARGANDOÑA PICAZO, Javier, 13071 Ciudad Real (ES); PRESA, Silvia, 13071 Ciudad Real (ES); RAMBLA, Jose Luis, 13071 Ciudad Real (ES)
(74) Representative: Balder IP Law, S.L.
(86) International application number: PCT/EP2022/075670
(87) International publication number: WO 2023/041661

(56) References cited:
- WO-A1-2016/012968
- WO-A2-2013/021261
- ES-A1- 2 701 354
- S. FRUSCIANTE ET AL: "Novel carotenoid cleavage dioxygenase catalyzes the first dedicated step in saffron crocin biosynthesis", PROCEEDINGS OF THE NATIONAL ACADEMY OF SCIENCES, vol. 111, no. 33, 5 August 2014 (2014-08-05), pages 12246 - 12251, XP055208496, ISSN: 0027-8424, DOI: 10.1073/pnas.1404629111
- MARTÍ MARICARMEN ET AL: "Efficient production of saffron crocins and picrocrocin in Nicotiana benthamiana using a virus-driven system", METABOLIC ENGINEERING, vol. 61, 1 September 2020 (2020-09-01), AMSTERDAM, NL, pages 238 - 250, XP055895215, ISSN: 1096-7176, DOI: 10.1016/j.ymben.2020.06.009
- GIANFRANCO DIRETTO ET AL: "UGT709G1: a novel uridine diphosphate glycosyltransferase involved in the biosynthesis of picrocrocin, the precursor of safranal in saffron ( Crocus sativus )", NEW PHYTOLOGIST, 1 July 2019 (2019-07-01), GB, XP055621925, ISSN: 0028-646X, DOI: 10.1111/nph.16079
- LIU TAOLI ET AL: "Prospects and progress on crocin biosynthetic pathway and metabolic engineering", vol. 18, 29 October 2020 (2020-10-29), Sweden, pages 3278 - 3286, XP055895232, ISSN: 2001-0370, Retrieved from the Internet <URL:http://dx.doi.org/10.1016/j.csbj.2020.10.019> DOI: 10.1016/j.csbj.2020.10.019
- AHRAZEM OUSSAMA ET AL: "Engineering high levels of saffron apocarotenoids in tomato", HORTICULTURE RESEARCH, vol. 9, 5 January 2022 (2022-01-05), pages uhac074, XP093008010, ISSN: 2662-6810, Retrieved from the Internet <URL:https://academic.oup.com/hr/article-pdf/doi/10.1093/hr/uhac074/43913591/uhac074.pdf> DOI: 10.1093/hr/uhac074

## Description

### TECHNICAL FIELD

The invention disclosed herein relates generally to the field of genetic engineering. Particularly, the invention disclosed herein provides methods and materials for recombinantly producing apocarotenoids from transgenic plants, preferably, plants from the *Solananum* genus, and more preferably, tomato and potato plants, expressing heterologous genes from *Crocus sativus,* the saffron plant, under the control of tuber-, fruit- and/or constitutive-promoters. Moreover, the present disclosure also relates to the genetic constructs comprising the heterologous genes, plasmid or vectors comprising the same, their use for the expression in plants, preferably in *Solanaceous* plants, and more preferably in tomatoes and potatoes, and to a method for obtaining such genetically modified plants, which express the genetic constructs mentioned above, and which have a high concentration of apocarotenoids products, in particular crocins and picrocrocins. Furthermore, the present disclosure also refers to the food products obtained from transgenic plants, preferably potatoes, potatoes-based-products, tomatoes and/or tomato-based products and the uses thereof as nutraceutical and pharmaceutical compositions, preferably for the treatment of neurodegenerative diseases such as Alzheimer's disease.

### STATE OF THE ART

Carotenoid biosynthesis in plants is catalyzed by nuclear-encoded enzymes that are imported to the plastids. Carotenoids are generated from five-carbon isoprenoids, isopentenyl diphosphate (IPP), and dimethylallyl diphosphate (DMAPP). The all-trans-lycopene is the major carotenoid produced in ripe tomatoes. Further all-trans-lycopene cyclization and hydroxylation reactions result in lutein and zeaxanthin compounds. In plants, zeaxanthin and lutein have several functions, including photoprotection, detoxification of reactive oxygen species and other radicals, and functional and structural integrity maintenance of biological membranes. Furthermore, zeaxanthin acts as a precursor of apocarotenoids as abscisic acid (ABA) in all plants, and crocins and picrocrocins in saffron, buddleja, and gardenia. Therefore, apocarotenoid compounds are derived from the oxidative cleavage of carotenoids in a reaction catalyzed by carotenoid cleavage dioxygenases (CCDs). The resulting products undergo different modifications to render the final active compounds with a broad range of biological functions in all living organisms. In plants, apocarotenoids are involved in plant fitness maintenance and in different aspects of development, with similar roles played in animals.

Crocins and picrocrocin are water-soluble apocarotenoids, and the major components of the saffron (*Crocus sativus*). These apocarotenoids accumulate in large amounts in the stigma of saffron, and they also display therapeutic properties, such as antioxidant, anticonvulsant, neuroprotective, anti-inflammatory, antidepressant, and antiproliferative activities, for which these characteristic apocarotenoids are partially responsible. The anti-oxidant and anti-inflammatory properties of apocarotenoids from saffron have been demonstrated in cardiovascular, digestive, and ocular diseases, and in leukemia. Furthermore, the effect of crocin-1 in Alzheimer disease has been also demonstrated in *in vitro* and *in vivo* studies as well as in a human trial with patients suffering from mild to moderate Alzheimer, wherein the patients that received 15 mg of saffron twice per day for 16 weeks showed a better cognitive function than patients who received the placebo. Saffron is also used as a spice and referred to as red gold due to its high economic value, which is a direct consequence of the specific characteristics of its cultivation and management. Crocins are responsible for the strong red color of saffron, and picrocrocins are associated with its bitter taste and is the precursor of safranal, the compound responsible for peculiar aroma and flavor.

These saffron apocarotenoids are derived from the cleavage of zeaxanthin at 7,8:7',8' positions, in a reaction catalyzed by a specific carotenoid cleavage dioxygenase enzyme (*Cs*CCD2L, GenBank Acc. ALM23547.1). Their water-solubility is provided by the glucose molecules added to their skeletons by the action of glucosyltransferases, *Cs*UGT2 (GenBank Acc. Num. AAP94878.1) and *Cs*UGT709G1 (GenBank Acc. Num. APU54677.1).

Between 120,000 and 200,000 flowers are needed to produce 1 kg of dried saffron stigma threads, which equates to 370-470 h of work. Consequently, the process is very labor-intensive as well as risky since it is highly dependent on environmental conditions, leading to high costs, and restricting the utilization of saffron by other industrial sectors. The saffron apocarotenoids has a global market size valued at 881.7 million USD in 2019.

In order to overcome the restrictions existing for obtaining saffron apocarotenoids and taking into account the advances of metabolic engineering and its progression into synthetic biology, the scientific community has extrapolated the synthesis of apocarotenoids from saffron to other hosts, looking for the low-cost production of these rare metabolites, such as saffron apocarotenoids.

It is known that the use of the saffron *Cs*CCD2L enzyme resulted in a maximum accumulation of 1.22 mg/l, 15.70 mg/l, and 4.42 mg/l of crocetin in, respectively, *Saccharomyces cerevisiae* (Chai et al., Microb. Cell Factories, 2017; 16; pp: 54; Tan et al., J. Exp. Bot., 2019; 70, pp: 4819-4834) and *Escherichia coli* (Wang et al., Microb. Cell Factories, 2019;18). In a subsequent study aimed to confirm, in planta, the role of a novel UGT in picrocrocin biosynthesis (Diretto et al., New Phytol., 2019; 224; pp: 725-740), *Nicotiana benthamiana* leaves were transiently transformed, via *Agrobacterium tumefaciens,* with *Cs*CCD2L, alone or in combination with UGT709G1, which led to the production of 30.5 µg/g dry weight of crocins, with a glycosylation degree ranging from 1 to 4 (crocin 1-4). In addition, Marti, M. *et al.,* using a virus-driven expression of *Cs*CCD2L in adult *N. benthamiana* plants demonstrated that *Cs*CCD2L expression alone is sufficient for significant crocin accumulation in transiently transformed tobacco (up to 2 mg/g dry weight (DW)) (Martí, M. et al. Metabolic Engineering. 2020; 61: 238-250). Stable transformation in *Nicotiana glauca* and *N. tabacum* expressing CsCCD2L alone, allowed the accumulation of 400 µg/g DW and 36 µg/g DW respectively (Ahrazem et al. Frontiers in Plant Science. 2022; 13:861140). Although promising, these results cannot be considered efficient and sustainable in the context of the development of an industrial system for the production of the saffron high-value apocarotenoids.

There is a constant need to make available further improved methods for increasing the production of saffron apocarotenoids, in particular crocins and picrocrocins, in alternatives heterologous systems.

### DESCRIPTION OF THE INVENTION

The present disclosure demonstrates that by engineering plants, preferably plants which belong to *Solananum* genus, and more preferably tomato and potato plants, in whose fruit and tubers in the case of potatoes, in this case tomato fruits, three saffron-specific genes involved in the biosynthesis of apocarotenoids in the crocus stigmas, are expressed, thus achieving the biosynthesis and accumulation of high levels of crocins, picrocrocin, HTTC and safranal in said fruits and tubers.

The pathway for crocins and picrocrocin biosynthesis was introduced into tomato and potato using tuber-, fruit-specific and constitutive promoters. Specifically, the promoters pE8 and p2A11 which are fruit specific and CaMV p35S promoter which is a constitutive promoter, resulted in 12.48 mg/g of crocins and 2.92 mg/g of picrocrocin in the tomato DW, which has been the highest level reported for saffron apocarotenoids in heterologous systems as a result of transient or stable metabolic engineering, without compromising plant growth (see **Example 4, Table 8).** Additionally, in potatoes, the promoter used was the patatin promoter (pPat) and resulted in accumulation of crocins and picrocrocin reaching 360 µg/g and 800µg/g DW, respectively, in cv Desirée and 3.02mg/g DW and 5.90 mg/g in cv OIH15WT expressing the construct O6 (SEQ ID NO: 34) (see **Example 7, Table 11).** The strategy involved the transformation of the tomato plants with a first plasmid comprising the *CsCCD2L* gene sequence as set forth in SEQ ID NO: 1 (GenBank accession number KP887110.1) under control of a fruit-specific promoter, which encodes for the CsCCD2L protein as set forth in SEQ ID NO: 2 (GenBank accession number ALM23547.1) to produce crocetin dialdehyde and 2,6,6-trimethyl-4-hydroxy-1-carboxaldehyde-1-cyclohexene, and with two additional plasmids comprising one of them the *CsUGT709G1* gene sequence as set forth in SEQ ID NO: 5 (GenBank accession number KX385186.1) and the other one the CsUGT2 gene sequence as set forth in SEQ ID NO: 3 (GenBank accession number AY262037.1), under control of a fruit-specific promoter and/or a constitutive promoter, which encodes for the glycosyltransferases UGT709G1 comprising the sequence as set forth in SEQ ID NO: 6 (GenBank Acc. Num. APU54677.1), and CsUGT2 comprising the sequence as set forth in SEQ ID NO: 4 (GenBank Acc. Num. AAP94878.1), respectively, to produce picrocrocin and crocins. In potato a unique construct was used comprising the 3 genes under the control of the pPat promoter using two different varieties Desirée and OIH15WT, the last one accumulates high content of zeaxanthin. Additionally, the described approaches have the advantage of using stably transformed tomatoes and potatoes rather other host such as, *N. benthamiana* leaves which have to be repeatedly transiently transformed (Martí, M. et al. Metabolic Engineering. 2020, 61, 238-250), thus offering an easy and constantly available source of valuable apocarotenoids.

Therefore, the present disclosure demonstrates that by expressing the three genes *CsCCD2L* (SEQ ID NO: 1), Cs*UGT709G1* (SEQ ID NO: 5) and *CsUGT2* (SEQ ID NO: 3) under the control of the specific promoters mentioned herein, preferably fruit-, tubers- and/or constitutive-promoter, high levels of these valuable saffron apocarotenoids metabolites are obtained, especially in tomatoes fruits and also in potato tubers, indicating that these genes were sufficient to confer on tomato and potato, their ability to produce them. This strategy resulted in decreased lycopene levels in the tomato fruit **(Example 3 and Table 7)** and violaxanthin levels in cv. Desirée and zeaxanthin and violaxanthin in OIH15WT **(Example 8 and Table 13).** Despite this, zeaxanthin was still not a major carotenoid in the tomato fruit neither in cv. Desirée. This indicated that CsCCD2L was able to intercept the fruit metabolic flux to push it towards the production of apocarotenoids, with a marked reduction in the levels of lycopene, β-carotene, and α-carotene **(Table 7)** and lutein, violaxanthin and antheraxanthin in cv. Desirée and zeaxanthin in OIH15WT **(Table 13).**

Total antioxidant activity in the transgenic tomato and potato tubers-lines, with reduced liposoluble carotenoids and high water-soluble apocarotenoids, was much higher than in the wild-type plants (WT). It has been reported that saffron has higher antioxidant activity levels compared to tomato or potato, thus the engineered crops for crocins and picrocrocin production could substantially increase their hydrophilic antioxidant levels. In addition, the protective effects of crocins against chronic stress-induced oxidative damage and their anti-inflammatory effects have been widely reported, and their presence will help to enhance the health properties of these tomatoes, which can be consumed either fresh or as processed products such as juices, soups, and sauces. In addition, crocins and picrocrocins can also simply be extracted from the pressed juice of the tomato or from the potato tubers and used for the affordable production of these compounds as pharmaceuticals in medicine or as colorants for the feed, food, and beverage industries.

Tomato (*Solanum lycopersicum*) is a popular and highly consumed food worldwide, which is used as a model crop for biotechnological applications. In this study, tomato was selected for the introduction of the saffron pathway because the fruit accumulates high levels of carotenoids as substrates, and there are well established downstream processes, including concentrates, juices, for human consumption. In addition, many commercially produced tomatoes are widely viewed as having poor taste, and their flavor is a major source of consumer dissatisfaction. The apocarotenoids of saffron provide the characteristic flavor and aroma of the saffron spice, and their solubility will enhance the nutraceutical properties of tomato juice. On the other hand, potato (*Solanum tuberosum* L.) is a commercially important crop throughout the world both for fresh and processed food industries. In addition to being the largest vegetative-propagated crop worldwide, potato tubers have become a staple in those parts of the world where there is an increasing pressure on limited land, and an increasing demand for food, because this plant is a short season crop, can be stored for long periods of time, and requires minimum inputs. Since it is grown in a wide range of climates, the tuber potato represents an excellent plant candidate to be used as a reservoir for producing natural metabolites. Thus, potato tubers with crocins display an intense yellow color and provide an increase in antioxidant activity.

Tomato fruits and potato tubers have been revealed to be good platforms for the production of the characteristic apocarotenoids of saffron as it is shown in the present disclosure. In ripening tomato fruits, the carotenoid pathway is highly active and for this reason, tomato fruits have previously been used to produce different exotic carotenoid compounds, such as ketocarotenoids. Although the biosynthesis of ketocarotenoids involved a single subcellular location in the cell, the plastid, the pathway for crocins and picrocrocin is more challenging, as it requires the participation of two main compartments, the plastid (the source) and the vacuole (the sink), where crocins accumulate. The central vacuole, functions as a storage compartment of hydrophilic secondary metabolites, allowing for the accumulation of huge amounts of metabolites, and thus offering an attractive compartment for the accumulation of polar secondary metabolites of interest. Comparison of our data with the production of other metabolites in tomatoes, such as ketocarotenoids (3 mg/g DW) (Nogueira, M. et al. Proceedings of the National Academy of Sciences of the United States of America. 2017, 114, 10876-10881), anthocyanins (5mg/g DW) (Butelli, E. et al. Nature Biotechnology. 2008, 26, 1301-1308), resveratrol (5-6 mg/g DW) (Zhang, Y. et al. Nature Communications. 2015, 6, 8635), and betacyanin (50 mg/L) in tomato juice (Polturak, G. et al. Proc Natl Acad Sci U S A. 2017, 114, 9062-9067), demonstrates the feasibility of engineering economically important metabolic pathways in tomato fruits with good yields for compound interests. It was estimated that the saffron apocarotenoids produced in these tomato fruits could provide an approximate 150-fold cost saving, as presently the production costs for saffron spice are in the range of 3,000- 6,000 USD per kilogram, assuming a production cost for tomato material containing a kilogram of saffron apocarotenoids of around 32 USD.

Comparison of the data shown in the present document with the production of other metabolites of interest in potato tubers, revealed the successful outcome of this apocarotenoid metabolic engineering, indeed, potato lines accumulating ketocarotenoid expressing the algal bkt1 gene, encoding a β-ketolase, showed an amount of 14 µg/g DW (Morris et al. Metabolic engineering (2006) 8(3):253-63.), whereas developing and mature tubers of transgenic crtB and crtB+crtl+crtY Desirée lines reached, c. 11 and 47 µg/g DW of β-carotene, respectively (Ducreux et al. Journal of experimental botany (2005) 56(409):81-9, Diretto G et al. PLoS One (2007) 2(4):e350., Diretto et al. Plant physiology (2010) 154(2):899-912.); while tubers overexpressing the Orange (Or) gene, involved in chromoplast differentiation accumulate up to 30 µg/g DW of β-carotene after 5 months storage in cold rooms (Li et al. Molecular plant (2012) 5(2):339-52); and, more recently, stable expression of CrtRb2 and PSY2 in transgenic lines increased the total carotenoid reaching c. 25 µg/g DW (Pasare et al. Protoplasma (2013) 250(6):1381-92).

Overall, this study demonstrates the potential and feasibility of using different species from *Solanum* genus, specially tomatoes (*Solanum lycopersicum*) and potatoes (*Solanum tuberosum*) as biotechnological tools for the simultaneous production of pro-nutritional lipophilic and rare high-valuable hydrophilic apocarotenoid compounds, such as crocins, picocrocins, HTTC and safranal, through a combinatorial approach in which by expressing the three genes *CsCCD2L* (SEQ ID NO: 1), *CsUGT2* (SEQ ID NO: 3) and *UGT709G1* (SEQ ID NO: 5) from saffron (*C*. *sativus*) under the control of the specific promoters, high levels of these valuable metabolites are obtained in the heterologous system, indicating that these three genes are sufficient to confer on tomato and potato, its ability to produce them. Thus, the tomatoes, potatoes, as well as the tomatoes-based products such as ketchup, a tomato-based sauce, pizza sauce, tomato soup or tomato juice and potatoes-based products such are potato flakes, potato chips, or potato flour, are therefore useful as nutraceuticals and medicaments. Preferably, the medicaments are pharmaceutical compositions for use as medicaments, and more preferably for use in the prevention and/or treatment of neurodegenerative diseases, such as Alzheimer Diseases.

Thus in a first aspect, the present disclosure provides a DNA construct, hereinafter the DNA construct of the invention, comprising nucleotide sequences encoding for a combination of three proteins for apocarotenoids compounds biosynthesis, preferably crocins, crocetins, picrocrocin, HTTC and safranal, selected from UGT2, UGT709G1 and CCD2L, or a functionally equivalent sequence thereof , which preferably belong to genus Crocus, and preferably to the species selected from the list consisting of: *Crocus sativus, Crocus angustifolius, Crocus ancyrensis, Crocus chrysanthus, Crocus pulchellus, Crocus sieberii y Crocus speciosus, more preferably to C. sativus,* wherein the amino acid sequences for UGT2, UGT709G1 and CCD2L are selected from an amino acid sequence at least 90%, 91%, 92%, 93%, 94%,95%, 96%, 97%, 98% or 99% identical to the sequences selected from the list consisting of SEQ ID NO: 4, SEQ ID NO: 6 and SEQ ID NO: 2 respectively, wherein each nucleotide sequences being operably linked to a promoter selected from a tuber specific promoter, a fruit specific promoter or a constitutive promoter, and wherein the nucleotide sequence encoding for the CCD2L is operably linked to a fruit- or tuber-specific promoter.

In a second aspect, the present disclosure provides a method for increasing the levels of apocarotenoids compounds in a plant, hereinafter the method for increasing the levels of apocarotenoids compounds of the invention, wherein the apocarotenoids compounds are preferably crocins, picrocrocins, HTTC and safranal in a plant, wherein the method comprising introducing and expressing the DNA construct as described herein into the plant.

In a third aspect, the present disclosure provides a method for producing a transgenic plant having increased levels of apocarotenoids compounds, hereinafter the method for producing a transgenic plant of the invention, wherein the apocarotenoids compounds are preferably crocins, picrocrocins, HTTC and safranal, compared to the corresponding wild-type plant, wherein the method comprising introducing and expressing the DNA construct as disclosed herein into the plant.

In a fourth aspect, the present disclosure provides transgenic plants, hereinafter the transgenic plants of the invention, which comprise in their genome the DNA construct as disclosed herein, wherein the plant accumulates increased levels of apocarotenoids compounds, preferably crocins, picrocrocins, HTTC and safranal compared to the corresponding wild-type plant.

In a fifth aspect, the present disclosure provides seeds, fruits, tubers, progeny and hybrids of the transgenic plant of the invention, hereinafter the seeds, fruits, tubers, progeny and hybrids of the invention.

In a sixth aspect, the present disclosure provides a food product prepared by the method for increasing the levels of apocarotenoids of the invention, wherein the food product having enhanced apocarotenoids compounds, preferably crocins, picrocrocins, HTTC and safranal levels.

In a seventh aspect, the present disclosure provides a pharmaceutical or nutraceutical composition comprising the food product of the invention, a fragment or an extract thereof, and a pharmaceutical or nutraceutical acceptable carrier.

In an eighth aspect, the present disclosure provides a pharmaceutical composition of the invention for use as medicine.

In a nineth aspect, the present disclosure provides a pharmaceutical composition of the invention for use as medicine for the prevention and/or treatment of neurodegenerative diseases, preferably Alzheimer's disease.

### BRIEF DESCRIPTION OF THE DRAWINGS

To complete the description and in order to provide for a better understanding of the invention, a set of drawings is provided. Said drawings form an integral part of the description and illustrate aspects and embodiments disclosed herein, which should not be interpreted as restricting the scope of the invention, but just as an example of how the invention can be carried out. The drawings comprise the following figures:

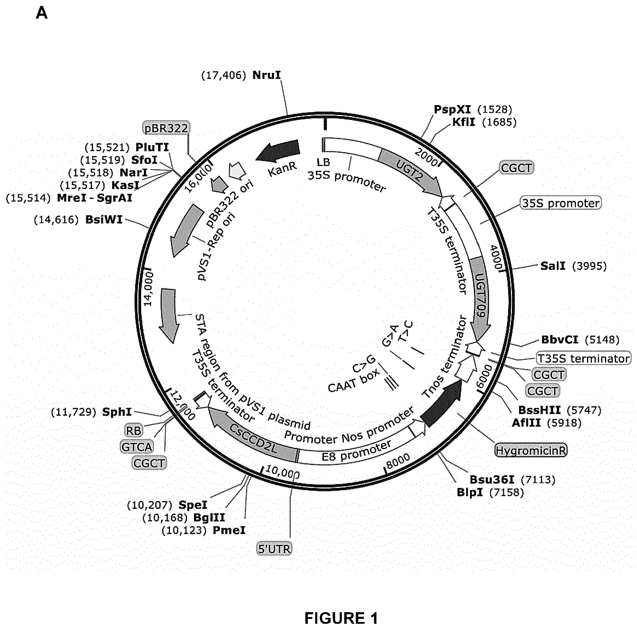

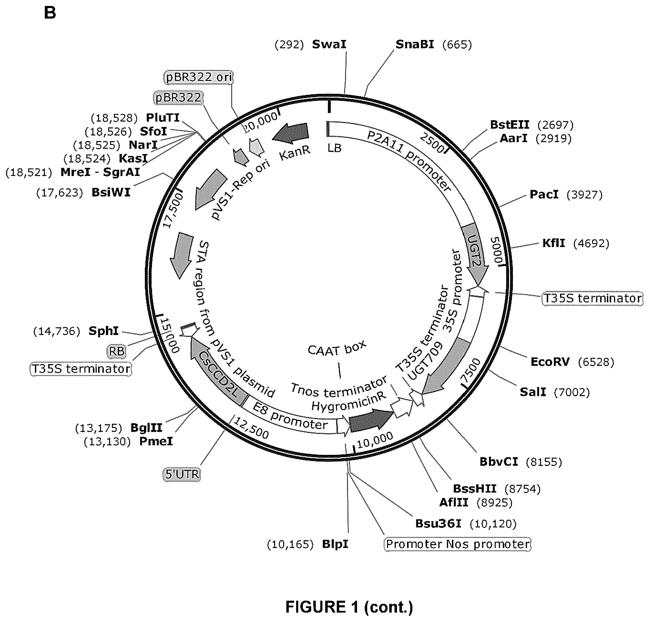

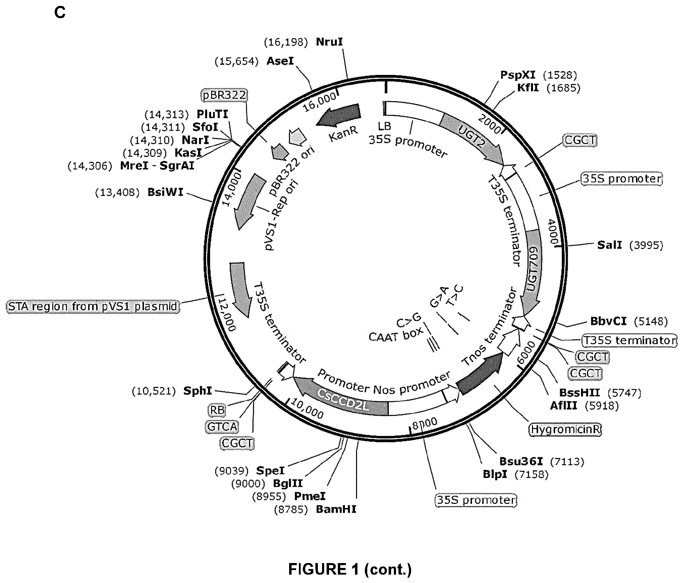

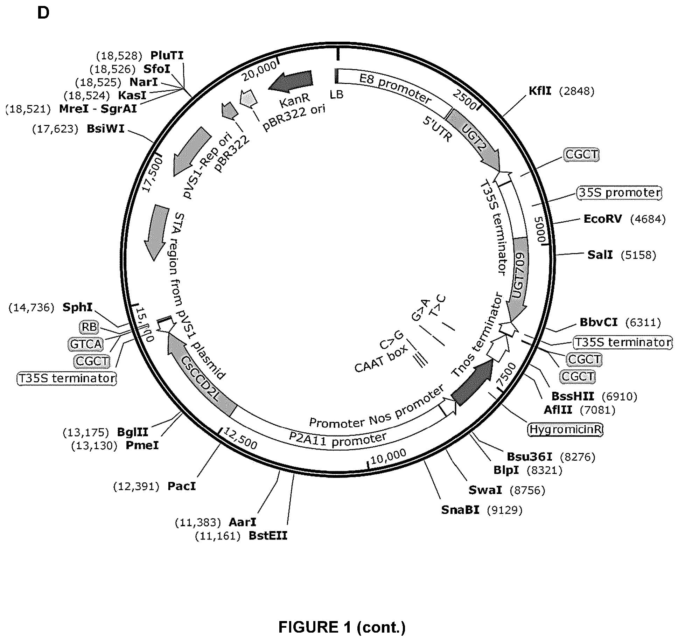

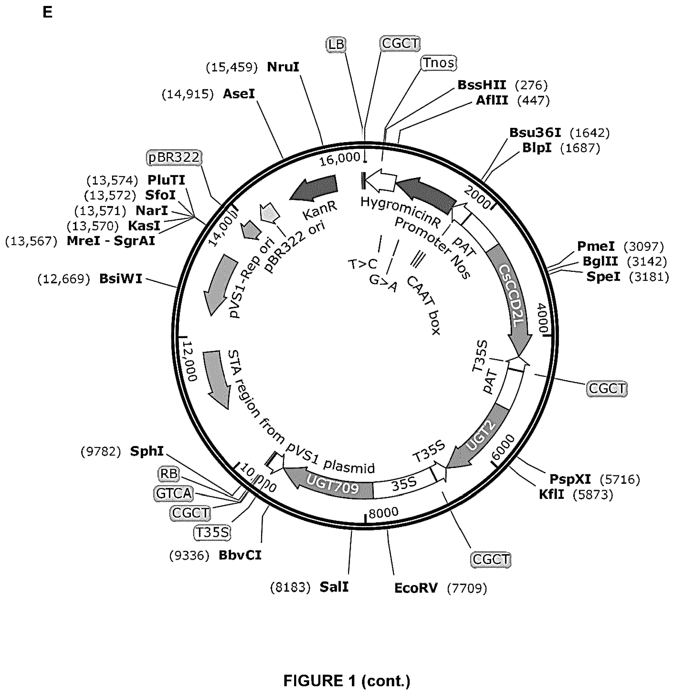

**Figure 1****.** Schematic representation of the four constructs **A)** O1 (pDGB3α1[p35SUGT2T35S-p35SUGT709T35S-pE8CCD2LT35S-pNos-Hyg-T35S]) (SEQ ID NO: 21), **B)** O2 (pDGB3α1[p2A11SUGT2T35S-p35SUGT709T35S-pE8CCD2LT35S-pNos-Hyg-T35S]) (SEQ ID NO: 22), **C)** O3 (pDGB3α1:[p35SUGT2T35S-p35SUGT709T35S-p35SCCD2LT35S-pNos-Hyg-T35S]) (SEQ ID NO: 23), **D)** O4 (pDGB3α1[pE8UGT2T35S-p35SUGT709T35S-p2A11CCD2LT35S-pNos-Hyg-T35S]) (SEQ ID NO: 24) used to transform tomato plants and one construct E) O6 (pDGB3α2[pPatatinGT2T35S-pPatatinUGT709T35S-pPatatinCCD2LT35S-pNos-Hyg-T35S]) (SEQ ID NO: 34) used to transform potato plants.

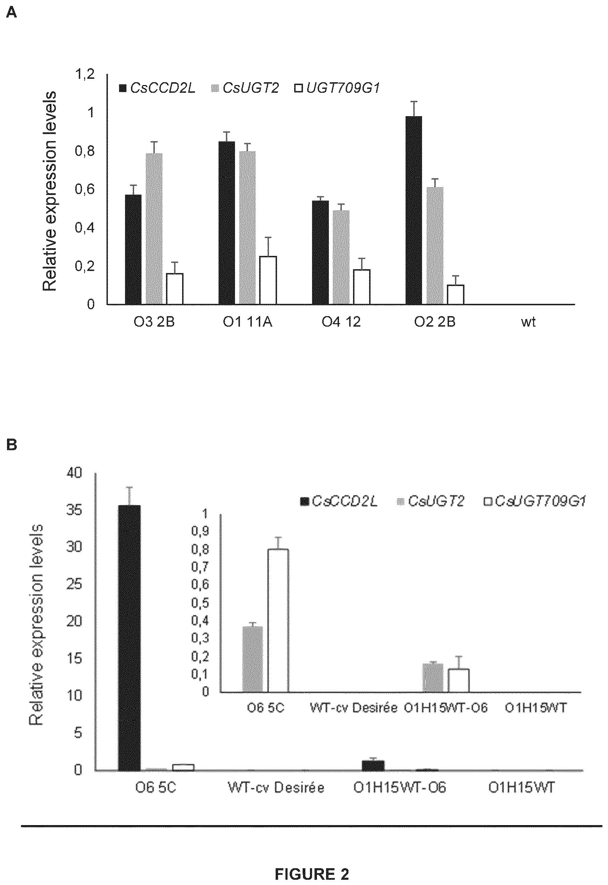

**Figure 2****.** Relative expression levels of *CsCCD2L, CsUGT2* and *CsUGT709G1* genes of **A)** the transgenes in wild-type (WT) and transgenic tomato ripe fruits (O3 2B, O1 11A, O4 12 AND O2 2B) and **B)** the transgenes in wild-type (WT-cv Desirée and OIH15WT) and potato tubers (O6 5C and OIH15WT-O6) obtained by RT-qPCR. In part **B** of this an inset showing a magnification of the original data has been included.

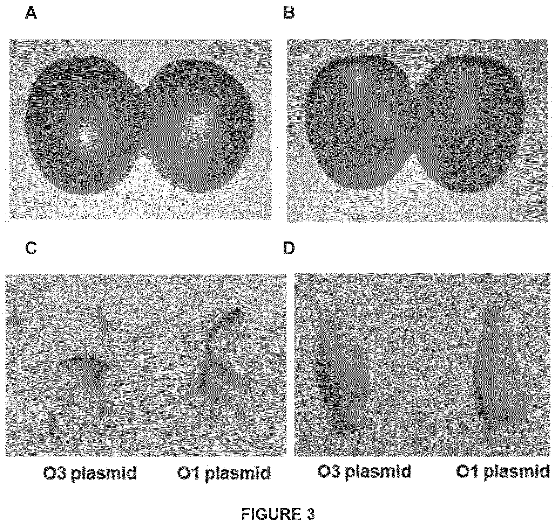

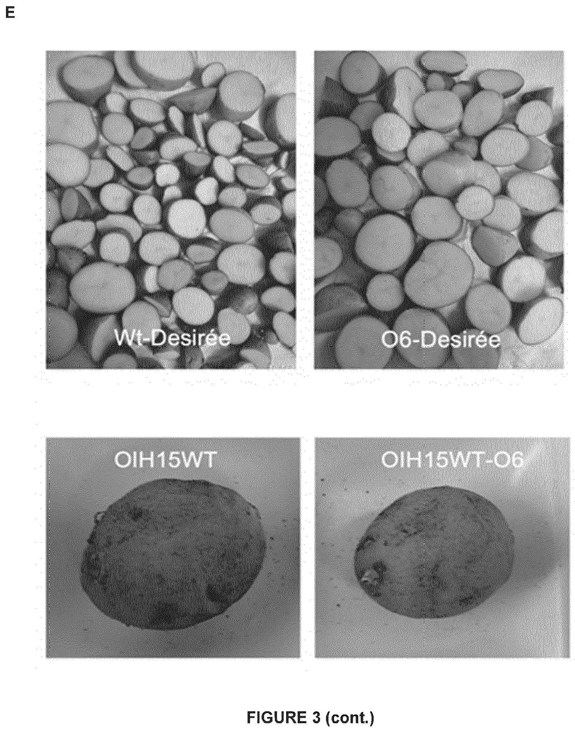

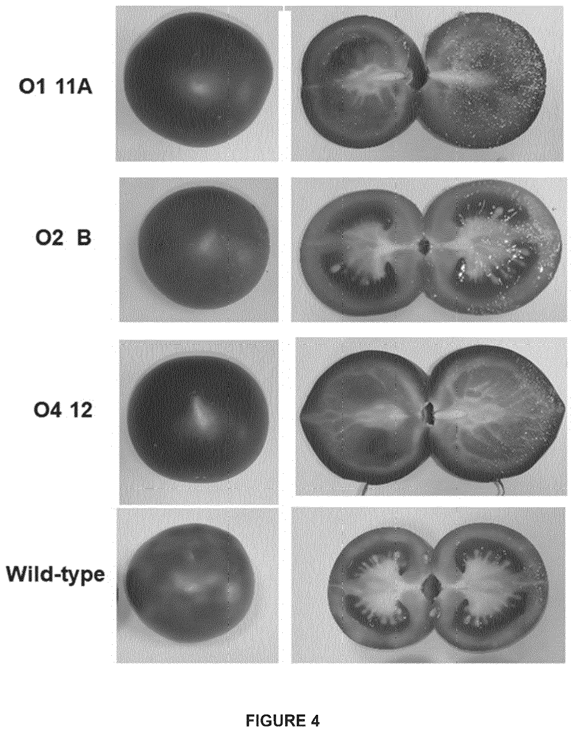

**Figure 3****.** Phenotype of ripe fruits and flowers of transgenic line engineered with the O3 plasmid. **(A)** and **(B):** view of the fruit with O3 plasmid outside and inside, respectively. **(C).** Flowers of transgenic lines transformed with O3 and O1 plasmids, respectively. **(D)** Dissected anthers of O3 and O1 transgenic lines. O1 plasmid: 35SUGT2-35SUGT709G1-E8CCD2L (SEQ ID NO: 21), and O3 plasmid: 35SUGT2-35SUGT709G1-35SCCD2L (SEQ ID NO: 23). Phenotypes of WT, and O1 fruits are shown in Figure 4. **(E)** Potato WT and transgenic line engineered with the O6 plasmid
**Figure 4****.** Phenotype of WT and transgenic ripe tomatoes carrying O1 (SEQ ID NO: 21), 02 (SESQ ID NO: 22) and O4 (SEQ ID NO: 24) constructs.

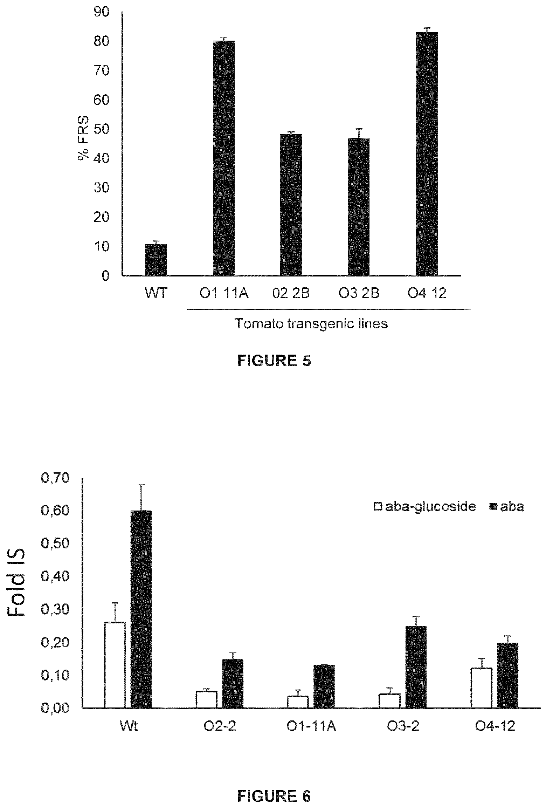

**Figure 5****.** Fruit antioxidant activity measured as percentage of Free radical scavenging (FRS) activity was analysed. Analysis of lipophilic antioxidant activity in ripe tomato fruit from WT and tomato transgenic lines by scavenging of 1.1-diphenyl-2-picrylhydrazyl (DPPH). Results are presented as percentage of radical scavenging capacity for extracts of WT and transgenic tomato lines. Values are the average of three replicates.
**Figure 6****.** Relative levels of abscisic acid (ABA) metabolites in WT and transgenic fruits (O2-2, O1-11A, O3-2 AND O4-12).

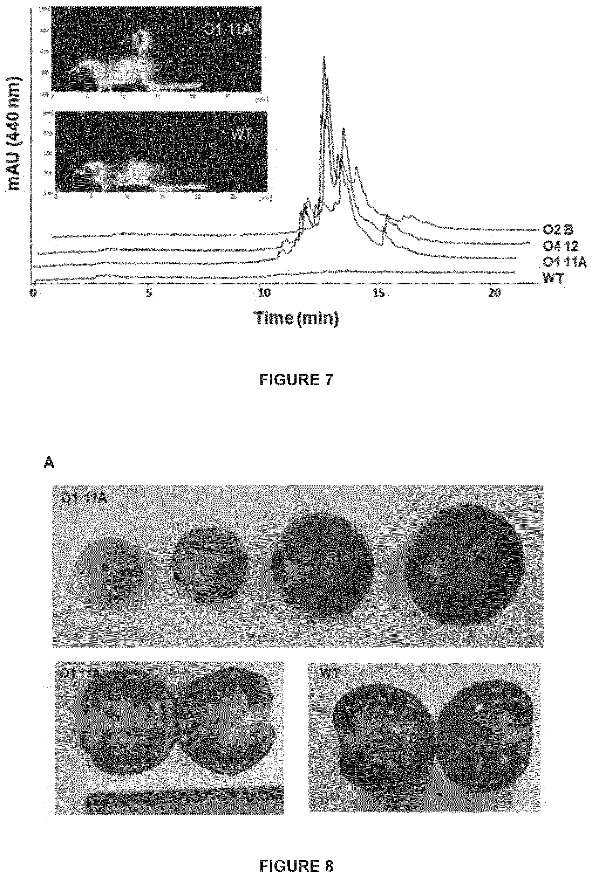

**Figure 7****.** Crocin and picrocrocin analyses in the fruits and serum juices of the WT and selected transgenic lines (O2 B, O4 12 and O1 11A). Representative chromatograms at 440 nm for the serum juice from the WT and transgenic lines. Inset: 2D chromatogram plots of the WT and O1 11A transgenic serum juices.

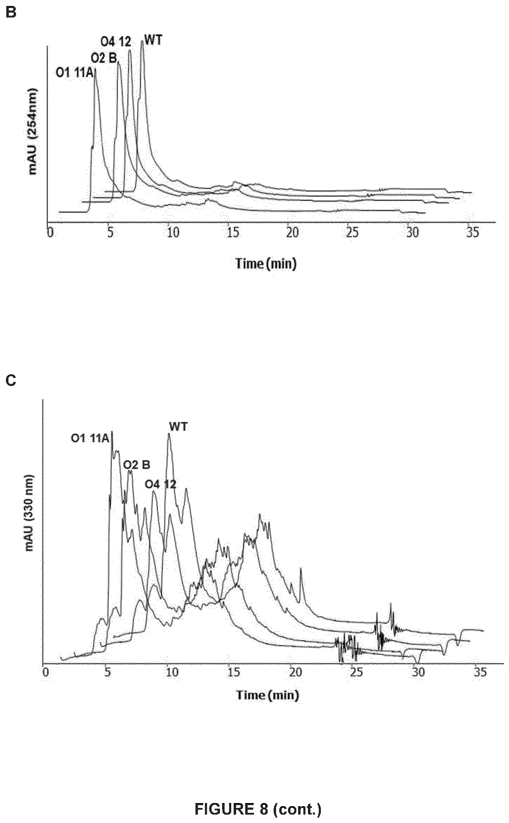

**Figure 8****.** Analyses of the serum juice of ripe WT and transgenic tomato lines. **A.** The crocins accumulation in transgenic fruits confer an orange coloration to the inside of the tomato fruit. **B.** Representative HPLC-DAD chromatograms of serum juice at 254 nm for the detection of flavonoids. **C.** Representative HPLC-DAD chromatograms of serum juice at 330 nm for the detection of phenolic compounds.

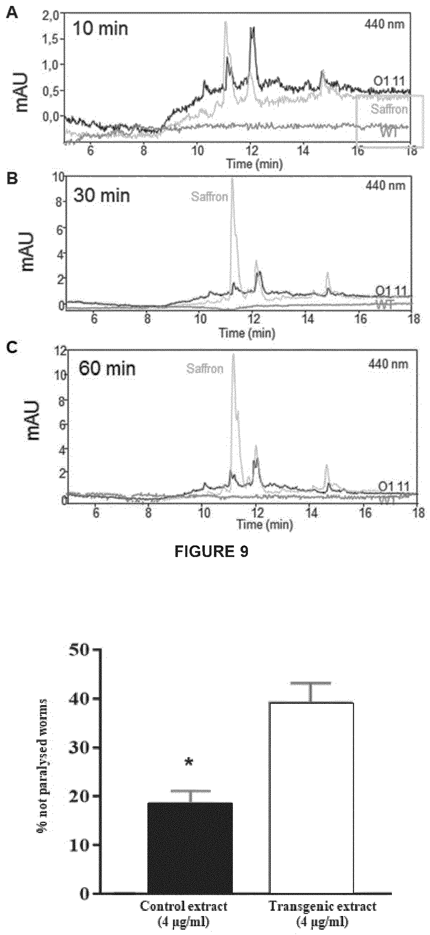

**Figure 9****.** Pigment diffusion experiment using dry intact or powder tomato ripe fruits and saffron stigma. **A, B and C,** shown HPLC-DAD chromatograms at 440 nm for crocins detection in water at different time-points.
**Figure 10****.** Effects on the paralysis induced in C. elegans CL4176 by the different extracts of wild-type (control) and transgenic tomato. * p < 0.05

### DETAILED DESCRIPTION OF THE INVENTION.

The following definitions and methods are provided to better define the present invention and to guide those of ordinary skill in the art in the practice of the present invention. Unless otherwise noted, terms are to be understood according to conventional usage by those of ordinary skill in the relevant art.

The practice of the present invention will employ, unless otherwise indicated, conventional techniques of botany, microbiology, tissue culture, molecular biology, chemistry, biochemistry and recombinant DNA technology, bioinformatics which are within the skill of the art. Such techniques are explained fully in the literature.

As used herein, the words "nucleic acid", "nucleic acid sequence", "nucleotide", "nucleic acid molecule" or "polynucleotide" are intended to include DNA molecules (e.g., cDNA or genomic DNA), RNA molecules (e.g., mRNA), naturally occurring, mutated, synthetic DNA or RNA molecules, and analogues of the DNA or RNA generated using nucleotide analogues. It can be single- stranded or double-stranded. Such nucleic acids or polynucleotides include, but are not limited to, coding sequences of structural genes, anti-sense sequences, and non-coding regulatory sequences that do not encode mRNAs or protein products. These terms also encompass a gene. The term "gene" or "gene sequence" is used broadly to refer to a DNA nucleic acid associated with a biological function. Thus, genes may include introns and exons as in the genomic sequence, or may comprise only a coding sequence as in cDNAs, and/or may include cDNAs in combination with regulatory sequences. In one embodiment, cDNA is preferred. Thus, in all of the aspects described herein that use nucleic acids, cDNA can be used unless otherwise specified.

The terms "peptide", "polypeptide" and "protein" are used interchangeably herein and refer to amino acids in a polymeric form of any length, linked together by peptide bonds.

The term "DNA construct" refers to a DNA molecule/s not normally associated in nature, capable of plant genomic integration, comprising one or more transgene DNA sequences that have been linked in a functionally operative manner using well-known recombinant DNA techniques. A "plurality" of DNA constructs refers to two or more. The present invention is based on the unexpected finding that levels of apocarotenoids, preferably, crocins, picrocrocins, HTCC and safranal are found in the transgenic plants of the invention.

"Promoter" refers to a nucleic acid sequence located upstream or 5' to a translational start codon of an open reading frame (or protein-coding region) of a gene and that is involved in recognition and binding of RNA polymerase II and other proteins (transacting transcription factors) to initiate transcription. "Constitutive promoters" are functional in most or all tissues of a plant throughout plant development. Tissue-, organ- or cell-specific promoters are expressed only or predominantly in a particular tissue, organ, or cell type, respectively. Thus, a "fruit promoter" is a native or non-native promoter that is functional in fruits cells, and a "tuber promoter" is a native or non-native promoter that is functional in tuber cells. Rather than being expressed "specifically" in a given tissue, organ, or cell type, a promoter may display "enhanced" expression, i.e., a higher level of expression, in one part (e.g., cell type, tissue, or organ) of the plant compared to other parts of the plant.

"Operably linked to one or more promoters" means the gene, or DNA sequence, is positioned or connected to the promoter in such a way to ensure its functioning. The promoter is any sequence sufficient to allow the DNA to be transcribed. After the gene and promoter sequences are joined, upon activation of the promoter, the gene will be expressed.

"Heterologous" sequence refers to a sequence which originates from a foreign source or species or, if from the same source, is modified from its original form.

"Recombinant" nucleic acid is made by an artificial combination of two or more otherwise separated segments of sequences, e.g., by chemical synthesis or by the manipulation of isolated segments of nucleic acids by genetic engineering techniques. Techniques for nucleic-acid manipulation are well-known in the art.

The terms "transformed," "transfected," or "transgenic" refers to a cell, tissue, organ, or organism into which has been introduced a foreign nucleic acid, such as a recombinant construct. Preferably, the introduced nucleic acid is integrated into the genomic DNA of the recipient cell, tissue, organ or organism such that the introduced nucleic acid is inherited by subsequent progeny. A "transgenic" or "transformed" cell or organism also includes progeny of the cell or organism and progeny produced from a breeding program employing such a "transgenic" plant as a parent in a cross and exhibiting an altered phenotype resulting from the presence of a recombinant construct or construct.

In certain embodiments, a transgenic plant for the purposes of the invention is thus understood as meaning, as above, that the nucleic acids used in the method of the invention are not at their natural locus in the genome of said plant, it being possible for the nucleic acids to be expressed homologously or heterologously. Thus, the plant expresses a transgene. Transgenic is preferably understood as meaning the expression of the nucleic acids according to the invention at an unnatural locus in the genome, i.e. homologous or, preferably, heterologous expression of the nucleic acids takes place. According to the invention, the transgene is stably integrated into the plant and the plant is preferably homozygous for the transgene.

The transgenic plant of the invention is non-naturally occurring. As used herein, the term "non-naturally occurring," when used in reference to a plant, means a seed plant that has been genetically modified by man. A transgenic plant of the invention, for example, is a non-naturally occurring plant that comprises exogenous nucleic acid molecules, such as a nucleic acid molecule encoding a heterologous UGT2, UGT709G1 and CCD2L gene product and, therefore, has been genetically modified by man.

The aspects of the invention involve recombination DNA technology and in preferred embodiments exclude embodiments that are solely based on generating plants by traditional breeding methods.

The inventors have surprisingly demonstrated that the heterologous expression of UGT2, UGT709G1 and CCD2L proteins which belong to Crocus family, preferably from *C. sativus,* in Solanaceous plants, preferably tomato and potato plants, leads to increased biosynthesis and accumulation of high levels of crocins, picrocrocin, HTTC and safranal in the fruits and tubers of said transgenic plants.

Thus, in a first aspect, the present disclosure relates to a nucleic acid or DNA construct comprising a nucleotide sequence encoding for a combination of three proteins for apocarotenoids compounds biosynthesis wherein the proteins are UGT2, UGT709G1 and CCD2L, or a functionally equivalent sequence thereof, which preferably belong to genus *Crocus L,*
wherein the amino acid sequences for UGT2, UGT709G1 and CCD2L are selected from an amino acid sequence at least 90%, 91%, 92%, 93%, 94%,95%, 96%, 97%, 98% or 99% identical to the sequences selected from the list consisting of SEQ ID NO: 4, SEQ ID NO: 6 and SEQ ID NO: 2 respectively,
wherein each nucleotide sequences being operably linked to a promoter selected from a fruit- or a tuber-specific promoter and/or a constitutive promoter.

In a preferred embodiment of the DNA construct of the invention, the nucleotide sequence encoding for the CCD2L is operably linked to a fruit-, or a tuber-specific promoter.

The genus Crocus L. (Iridaceae) comprises more than 88 different species of herbaceous perennials and geophytes, distributed throughout central and southeastern Europe, from Central Europe to Turkey, North Africa and from southwest Asia to Western China. By "Crocus L." it is understood a genus of herbaceous, perennial and geophite plants belonging to the Iridaceae family and which are characterized by presenting an underground reserve organ called corm. Examples, by way of illustration and not limitation, of species, subspecies and/or varieties of Crocus L. which are used within the scope of the invention are *C*. *sativus, C. ancyrensis, C. cancellatus, C. carpetanus, C. chrysanthus, C. nevadensis, C. serotinus, C. serotinus ssp. salzmanii, C. vernus var. "flower record", C. vernus var. "geel yellow", C. vernus "jeam d'arc", C. versicolor, C. speciosus, C. speciosus "albus", C. speciosus "Aitchinsonii", C. angustifolius, C. dalmaticus, C. pulchellus, C corsicus, C. kotschyanus, C. kotchyanus "albus", C. cartwrightianus, C. laevigatus "fontenayi"* and *C. ligusticus.*

In a preferred embodiment, the nucleotide sequences encoding for UGT2, CCD2L and UGT709G1 proteins belong to *C*. *sativus.*

A "CCD2L polypeptide", also known as carotenoid cleavage dioxygenase 2L, has the amino acid sequence of SEQ ID NO: 2 (GenBank ALM23547.1), encoded by the nucleotide sequence of SEQ ID NO: 1 (GenBank KP887110.1) and belong to *Crocus sativus.* In a preferred embodiment the CCD2L polypeptide has an amino acid sequence with an identity of at least 50%, more preferably 60%, 70%, 80%, 85%, 90%, 91%, 92%, 93%, 94%,95%, 96%, 97%, 98% and even more preferably 99% with the sequence SEQ ID NO: 2. In the same sense, the CCD2L nucleotide sequence has a nucleotide sequence with an of at least 50%, more preferably 60%, 70%, 80%, 85%, 90%, 91%, 92%, 93%, 94%,95%, 96%, 97%, 98% and even more preferably 99% with the sequence SEQ ID NO: 1.

A "UGT2 polypeptide", also known as Crocetin glucosyltransferase 2, has the amino acid sequence of SEQ ID NO: 4 (GenBank AAP94878.1), encoded by the nucleotide sequence of SEQ ID NO: 3 (GenBank AY262037.1) and belong to *Crocus sativus.* In a preferred embodiment the UGT2 polypeptide has an amino acid sequence with an identity of at least 50%, more preferably 60%, 70%, 80%, 85%, 90%, 91%, 92%, 93%, 94%,95%, 96%, 97%, 98% and even more preferably 99% with the sequence SEQ ID NO: 4. In the same sense, the UGT2 nucleotide sequence has an nucleotide sequence with an of at least 50%, more preferably 60%, 70%, 80%, 85%, 90%, 91%, 92%, 93%, 94%,95%, 96%, 97%, 98% and even more preferably 99% with the sequence SEQ ID NO: 3.

A "UGT709G1 polypeptide", has the amino acid sequence of SEQ ID NO: 6 (GenBank APU54677.1), encoded by the nucleotide sequence of SEQ ID NO: 5 (GenBank KX385186.1) and belong to *Crocus sativus.* In a preferred embodiment the UGT709G1 polypeptide has an amino acid sequence with an identity of at least 50%, more preferably 60%, 70%, 80%, 85%, 90%, 91%, 92%, 93%, 94%,95%, 96%, 97%, 98% and even more preferably 99% with the sequence SEQ ID NO: 6. In the same sense, the UGT709G1 nucleotide sequence has a nucleotide sequence with an of at least 50%, more preferably 60%, 70%, 80%, 85%, 90%, 91%, 92%, 93%, 94%,95%, 96%, 97%, 98% and even more preferably 99% with the sequence SEQ ID NO: 5.

The correspondence between the nucleotide sequence (s) of the putative sequence (s) and the sequence set forth in SEQ ID NO: 1 to 6 can be determined by methods known in the art. Sequence comparison methods are known in the state of the art, and include, but are not limited to, the BLAST or BLASTN program, and FASTA (Altschul et al., J. Mol. Biol. 215: 403-410 (1999).

The term "homology", as used herein, refers to the similarity between two structures due to a common evolutionary ancestry, and more specifically, to the similarity between two or more nucleotide or amino acid sequences in terms of percent nucleotide or amino acid positional identity, respectively, i.e., sequence similarity or identity. Homology also refers to the concept of similar functional properties among different nucleic acids or proteins
The term "identity", as used herein, refers to the proportion of identical nucleotides or amino acids between two nucleotide or amino acid sequences that are compared. Sequence comparison methods are known in the state of the art, and include, but are not limited to, the GAG program, including GAP (Devereux et al., Nucleic Acids Research 12: 287 (1984) Genetics Computer Group University of Wisconsin, Madison, (WI); BLAST, BLASTP or BLASTN, and FASTA (Altschul et al., J. Mol. Biol. 215: 403-410 (1999).

The term "functional equivalent sequence" as used herein, for example with reference to SEQ ID NO: 1 to 6, or any sequence mentioned herein, refers to a variant gene or peptide/protein sequence or part of the gene or peptide/protein sequence which retains the biological function of the full non-variant sequence. A functional equivalent sequence also comprises a variant of the gene of interest encoding a peptide which has sequence alterations that do not affect function of the resulting protein, for example in non-conserved residues. Also encompassed is a variant that is substantially identical, i.e. has only some sequence variations, for example in non-conserved residues, to the wild type sequences as shown herein and is biologically active. In this sense, functional equivalent sequences with reference to SEQ ID NO: 1 to 6, refers to a variant gene belonging to species different from *C*. *sativus,* such as for example, *C*, *angustifolius, C. ancyrensis, C. chrysanthus, C. pulchellus, C. sieberii and C. speciosus,* which comprises nucleotide sequences that encodes for apocarotenoids compounds biosynthesis and that are at least 90%, 91%, 92%, 93%, 94%,95%, 96%, 97%, 98% and even more preferably 99% with an of the sequences selected from SEQ ID NO: 1 to 6 of the present invention.

Thus, it is understood, as those skilled in the art will appreciate, that the aspects of the invention, including the methods and uses, encompass not only a nucleic acid or peptide as described herein, but also functional variants or homologues thereof that do not affect the biological activity and function of the resulting protein. Alterations in a nucleic acid sequence which result in the production of a different amino acid at a given site that do however not affect the functional properties of the encoded polypeptide, are well known in the art. For example, a codon for the amino acid alanine, a hydrophobic amino acid, may be substituted by a codon encoding another less hydrophobic residue, such as glycine, or a more hydrophobic residue, such as valine, leucine, or isoleucine. Similarly, changes which result in substitution of one negatively charged residue for another, such as aspartic acid for glutamic acid, or one positively charged residue for another, such as lysine for arginine, can also produce a functionally equivalent product. Each of the proposed modifications is well within the routine skill in the art, as is determination of retention of biological activity of the encoded products.

In another preferred embodiment of the DNA construct of the invention, the apocarotenoids compounds preferably synthetized are selected from the list consisting of: crocins, crocetins, picrocrocin, HTTC and safranal, among other.

In other preferred embodiment for the DNA construct of the invention, the CCD2L polypeptide (SEQ ID NO: 2) is encoded by the nucleotide sequence having at least 90%, 91%, 92%, 93%, 94%,95%, 96%, 97%, 98% and even more preferably 99% with the sequence SEQ ID NO: 1.

In other preferred embodiment for the DNA construct of the invention, the UGT2 polypeptide (SEQ ID NO: 4) is encoded by a nucleotide sequence having at least 90%, 91%, 92%, 93%, 94%,95%, 96%, 97%, 98% and even more preferably 99% with the sequence SEQ ID NO: 3.

In other preferred embodiment for the DNA construct of the invention, the UGT709G1 polypeptide (SEQ ID NO: 6) is encoded by the nucleotide sequence having at least 90%, 91%, 92%, 93%, 94%,95%, 96%, 97%, 98% and even more preferably 99% with the sequence SEQ ID NO: 5.

In other preferred embodiment for the DNA construct of the invention, the fruit specific promoter is selected from the list consisting of: tomato E8 promoter (SEQ ID NO: 7) and p2A11 (SEQ ID NO: 8) and combinations thereof.. In another preferred embodiment for the DNA construct of the invention, the tuber specific promoter is the patatin promoter pPat (SEQ ID NO: 33). In another preferred embodiment for the DNA construct of the invention, the constitutive promoter is the Cauliflower Mosaic Virus 35S promoter (SEQ ID NO: 9).

Preferably the DNA construct according to the invention is comprised within a vector, most suitably an expression vector adapted for expression in an appropriate host (plant) cell. It will be appreciated that any vector which is capable of producing a plant comprising the introduced DNA sequence will be sufficient. Suitable vectors are well known to those skilled in the art and are described in general technical references such as Pouwels et al, Cloning Vectors. A laboratory manual, Elsevier, Amsterdam (1986).

In another preferred embodiment for the DNA construct of the invention, the construct comprises the CCD2L nucleotide sequence operably linked to a fruit promoter which is preferably pE8, the UGT2 nucleotide sequence operably linked to a constitutive promoter which preferably is p35 and the UGT7091 nucleotide sequence operably linked to a constitutive promoter which is preferably p35S. In a more preferably embodiment for the DNA construct of the invention, the construct comprises the SEQ ID NO: 21 and is named in the present disclosure as O1.

In another preferred embodiment for the DNA construct of the invention, the construct comprises the CCD2L nucleotide sequence operably linked to a fruit promoter which is preferably pE8, the UGT2 nucleotide sequence operably linked to a fruit promoter which preferably is p2A11 and the UGT7091 nucleotide sequence operably linked to a constitutive promoter which is preferably p35S. In a more preferably embodiment for the DNA construct of the invention, the construct comprises the SEQ ID NO: 22 and is named in the present disclosure as O2.

In another preferred embodiment for the DNA construct of the invention, the construct comprises the CCD2L nucleotide sequence operably linked to a constitutive promoter which is preferably p35S, the UGT2 nucleotide sequence operably linked to a constitutive promoter which is preferably p35S and the UGT7091 nucleotide sequence is also operably linked to a constitutive promoter which is preferably p35S. In a more preferably embodiment for the DNA construct of the invention, the construct comprises the SEQ ID NO: 23 and is named in the present disclosure as O3.

In another preferred embodiment for the DNA construct of the invention, the construct comprises the CCD2L nucleotide sequence operably linked to a fruit promoter which is preferably pE8, the UGT2 nucleotide sequence operably linked to a fruit promoter which preferably is pE8 and the UGT7091 nucleotide sequence is operably linked to a constitutive promoter which is preferably p35S. In a more preferably embodiment for the DNA construct of the invention, the construct comprises the SEQ ID NO: 24 and is named in the present disclosure as O4.

In another preferred embodiment for the DNA construct of the invention, the construct comprises the CCD2L nucleotide sequence operably linked to a tuber specific promoter which is preferably pPat, the UGT2 nucleotide sequence operably linked tuber specific promoter which is preferably pPat and the UGT7091 nucleotide sequence is also operably linked to tuber specific promoter which is preferably pPat. In a more preferably embodiment for the DNA construct of the invention, the construct comprises the SEQ ID NO: 34 and is named in the present disclosure as O6.

In another aspect, the present disclosure also relates to the use of the DNA construct described above for increasing the levels of apocarotenoids compounds in a plant versus a wild-type plant not comprising the DNA construct of the invention.

The terms "increase", "improve" or "enhance" are interchangeably used herein and they refer to an increased, for example, by at least 2%, 3%, 4%, 5%, 6%, 7%, 8%, 9%, 10% or more, for example at least 15% or 20%, or 25%, 30%, 35%, 40% or 50% in comparison to a control or wild-type plant.

In another aspect, the present disclosure also relates to a method for increasing the levels of apocarotenoids compounds in a plant, wherein the method comprising introducing and expressing the DNA construct of the invention into the plant.

In a preferred embodiment for the method for increasing the levels of apocarotenoids of the present invention, it is characterized in that the apocarotenoids are selected from the list consisting of: crocins, picrocrocins, HTTC and safranal.

In another preferred embodiment for the method for increasing the levels of apocarotenoids of the present invention, it is characterized in that the plant belonging to the Solananum genus, preferably selected from the group consisting of tomato, potato, petunia, eggplant, tobacco and pepper, more preferably tomato, potato and petunia, and more preferably yet, tomato (*Solanum lycopersicum*) and potato (*Solanum tuberosum*).

In a more preferably embodiment for the method for increasing the levels of apocarotenoids of the present invention, it is characterized in that the apocarotenoids compounds, preferably crocins, picrocrocins, HTTC and safranal of said plants are increased in the harvestable parts of the transgenic plant, preferably in the tubers, fruits and seeds thereof.

In another aspect, the present disclosure also relates to a method for producing a transgenic plant having increased levels of apocarotenoids compounds compared to the corresponding wild-type plant, wherein the method comprising introducing and expressing the DNA construct of the present invention.

In one embodiment of the method for producing a transgenic plant of the invention, said plant, as mentioned above, belonging to the Solananum genus, preferably selected from the group consisting of tomato, potato, petunia, eggplant, tobacco and pepper, more preferably tomato, potato and petunia, and more preferably yet, tomato (*Solanum lycopersicum*) and potato (*Solanum tuberosum*).

In another preferred embodiment of the method for producing a transgenic plant of the invention, the apocarotenoids are selected from the list consisting of: crocins, picrocrocins, HTTC and safranal. In a more preferred embodiment, said apocarotenoids are increased in the harvestable parts of the transgenic plant, preferably in the fruits, tubers and seeds thereof.

Transformation techniques for introducing the DNA constructs according to the invention into host cells are well known in the art and include such methods as microinjection, using polyethylene glycol, electroporation, or high velocity ballistic penetration. A preferred method for use according to the present invention relies on Agrobacterium - mediated transformation.

The methods of the invention may also optionally comprise the steps of screening and selecting plants for those that comprise the DNA construct as above, have increased apocarotenoids compounds compared to a control or wild-type plant. Preferably, according to the methods described herein, the progeny plant is stably transformed and comprises the exogenous polynucleotide which is heritable as a fragment of DNA maintained in the plant cell and the method may include steps to verify that the construct is stably integrated. The method may also comprise the additional step of collecting the harvestable parts from the plants or from the selected progeny thereof.

In another aspect, the present disclosure also related to a plant obtained or obtainable by a method described herein.

In another aspect, the present disclosure also related to a transgenic plant which comprises in its genome the DNA construct disclosed herein, and wherein said plant accumulates increased levels of apocarotenoids compounds, preferably crocins, picrocrocins, HTTC and safranal compared to the corresponding wild-type plant.

In a preferred embodiment the transgenic plant of the invention belonging to Solananum genus, preferably is selected from the group consisting of tomato, potato, petunia, eggplant, tobacco and pepper, more preferably tomato, potato and petunia, and more preferably, tomato (*Solanum Iycopersicum*) and potato (*Solanum tuberosum*).

In a preferred embodiment, the transgenic plant of the invention it is characterized in that the increased levels of apocarotenoids are in the harvestable parts of the transgenic plant, preferably in the fruits, tubers and seeds.

The various aspects of the invention described herein clearly extend to any plant cell or any plant produced, obtained or obtainable by any of the methods described herein, and to all plant parts and propagules thereof unless otherwise specified. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The present disclosure also relates to the use of the transgenic plants according to the invention or desired parts thereof in the preparation of food products or pharmaceutical compositions.

In another aspect, the present disclosure also related to harvestable parts of the transgenic plants described above or a product derived therefrom.

In a preferred embodiment, the harvestable parts of the transgenic plants are selected from seeds, leaves, fruits, flowers, stems, roots, rhizomes, tubers and bulbs. More preferably, the harvestable parts are selected from seeds, tubers and fruits, and they are characterized by having increased levels of apocarotenoids disclosed herein as compared to the harvestable parts of wild-type plants.

The present disclosure also refers to products derived from harvestable parts of the transgenic plants of the invention, preferably directly derived, from a harvestable part of such a plant, such as dry pellets or powders, oil, fat and fatty acids, fiber or proteins.

The present disclosure also relates to food products and food supplements comprising preferably the harvestable parts of the transgenic plant of the invention, more preferably, tubers, fruits and/or seeds. The food products and food supplements have increased apocarotenoids compounds as disclosed herein, compared to food products or food supplements comprising harvestable parts of control or wild-type plants.

In a preferred embodiment the food product or food supplement is preferably a tomato, a potato, a tomato-based product and a potato-based product. In a more preferred embodiment, the tomato-based product is selected from the list consisting of ketchup, a tomato-based sauce, a pizza sauce, tomato soup or tomato juice, among others, and the potato-based product is selected from the list consisting of potato chips, potato flakes, or potato flour, among others.

In another aspect, the present disclosure also relates to a nutraceutical composition comprising the food product as disclosed herein, a fragment or an extract thereof, and a nutraceutical acceptable carrier.

As used herein, "nutraceutical composition" refers to a product isolated or purified from plant materials, and which is generally provided in formats not usually associated with foods. A nutraceutical composition has demonstrable physiological benefits for protection against neurodegenerative diseases, such as Alzheimer disease.

As used herein, "nutraceutical carrier" refers a suitable vehicle which is biocompatible and nutraceutically acceptable, and may comprise one or more solid, semi-solid or liquid diluents, excipients, flavours or encapsulating substances which are suitable for consumption.

In another aspect, the present disclosure also relates to a pharmaceutical composition comprising an effective amount of the food product as disclosed herein, a fragment or an extract thereof, and a pharmaceutical acceptable carrier and/or excipient.

As used herein, "effective amount" refers to an amount of a given compound that when administered to a mammal, preferably a human, is sufficient to produce prevention and/or treatment, as defined below, of a disease or pathological condition of interest in the mammal, preferably a human. The therapeutically effective amount will vary, for example, according to the age, body weight, general state of health, sex and diet of the patient; the mode and time of administration; the rate of excretion, the combination of drugs; the severity of the particular disorder or pathological condition; and the subject undergoing therapy, but can be determined by a specialist in the art according to his own knowledge.

The term "excipient" refers to a substance that helps the absorption of any of the components of the product of the invention, stabilizes these components or helps the preparation of the pharmaceutical composition in the sense of giving it consistency or providing flavors that make it more pleasant. Thus, the excipients could have the function of keeping the components together such as starches, sugars or cellulose, sweetening function, dye function, drug protection function such as to isolate it from air and/or moisture, function filling a tablet, capsule or any other form of presentation, a disintegrating function to facilitate the dissolution of the components and their absorption in the intestine, without excluding other types of excipients not mentioned in this paragraph.

The term "vehicle" or "carrier", is preferably an inert substance. The function of the vehicle is to facilitate the incorporation of other compounds, allow a better dosage and administration or give consistency and form to the pharmaceutical composition. Therefore, the carrier is a substance that is used to dilute any of the components of the pharmaceutical composition of the present invention to a given volume or weight; or that even without diluting said components it is capable of allowing a better dosage and administration or giving consistency and form to the medicine. It's vehicle is pharmaceutically acceptable. When the form of presentation is liquid, the pharmaceutically acceptable carrier is the diluent.

In addition, the excipient and the vehicle must be pharmacologically acceptable, that is, the excipient and the vehicle are allowed and evaluated so as not to cause damage to the organisms to which it is administered.

The pharmaceutical composition of the invention may comprise another active substance. In addition to the requirement of therapeutic efficacy, where said pharmaceutical composition may require the use of other therapeutic agents, there may be additional fundamental reasons that compel or strongly recommend the use of a combination of a compound of the invention and another therapeutic agent. The term "active principle" is any matter, whatever its origin, human, animal, plant, chemical or other, to which an appropriate activity is attributed to constitute a medicine.

In each case the form of presentation of the medicament will be adapted to the type of administration used, therefore, the composition of the present invention can be presented in the form of solutions or any other form of clinically permitted administration and in a therapeutically effective amount. The pharmaceutical composition of the invention can be formulated in solid, semi-solid, liquid or gaseous forms, such as tablet, capsule, powder, granule, ointment, solution, suppository, injectable, inhalant, gel, syrup, nebulizer, microsphere or aerosol, preferably in the form of a tablet, capsule, powder, granule, solution, suppository or syrup.

The above-mentioned compositions may be prepared using conventional methods, such as those described in the Pharmacopoeias of different countries and in other reference texts.

The compounds and compositions of the present invention can be used together with other medicaments in combination therapies. The other drugs may be part of the same composition or of a different composition, for administration at the same time or at different.

In another aspect, the present disclosure also relates to the pharmaceutical composition of the invention for use as medicine or medicament.

In another aspect, the present disclosure also relates to the pharmaceutical composition of the invention for use in the prevention and/or treatment of neurodegenerative diseases such as Alzheimer disease, or other forms of dementia such as Parkinson disease.

### EXAMPLES

Following are examples of the invention by means of assays carried out by the inventors, which evidence the effectiveness of the product of the invention. The following examples serve to illustrate the invention and must not be considered to limit the scope thereof.

### MATERIALS AND METHODS

### Plant material and growth conditions

Tomato (*S. lycopersicum* cv. Moneymaker (MM)) was used as the wild-type (WT) and the genetic background for all tomato plant transformations. Tomato fruits were collected at the ripe stage and pericarp tissues, once separated from the seeds and placenta, were cut into pieces, and immediately frozen in liquid nitrogen until further analyses.

Potatoes plants *(Solanum tuberosum* cv. Desirée and cv. OIH15WT) were grown from seed tubers or *in vitro*-propagated tissue culture plants in 10 cm diameter pots containing compost. Potatoes plants were raised in a greenhouse maintained at a day/night temperature of 20/15 °C. The maximum irradiance was approximately 10500 µmol m-2 s-1 and the mean day length was 16 h. Tubers were harvested from plants after flowering but prior to senescence.

### Plasmid construction

A series of plasmids were created with different promoter combinations to evaluate their ability to engineer the saffron apocarotenoid pathway in tomatoes and potatoes expressing the heterologous genes *CsCCD2L* gene sequence as set forth in SEQ ID NO: 1 (GenBank accession number KP887110.1), *CsUGT709G1* gene sequence as set forth in SEQ ID NO: 5 (GenBank accession number KX385186 ) and the *CsUGT2* gene sequence as set forth in SEQ ID NO: 3 (GenBank accession number AY262037.1), Three promoters, pE8 (SEQ ID NO: 7), p2A11 (SEQ ID NO: 8), and p35S (SEQ ID NO: 9), were selected to drive the expression of the saffron genes in tomato and one promoter pPat (SEQ ID NO: 33) to drive the expression of the saffron genes in potato **(****Figure 1****).** The Goldenbraid strategy was followed to construct the plasmids (Sarrion-Perdigones, A., et al. Methods Mol Biol. 2014, 1116, 133-151) using the primers listed in **Table 1** below.

**Table 1. Oligonucleotides used for plasmids construction.**

| **Primers** | **Sequence 5'-3'** | **SEQ ID NO:** |
|---|---|---|
| pUPD2-Dom-UGT2-F | gcgccgtctcgctcgaatgttgaacggcaacaaatgc | 10 |
| pUPD2-Dom-UGT2-R | gcgccgtctcgctcaaagcttaaactaaggaaattttggagtcat | 11 |
| pUPD2-Dom-CsCCD2-L-F1 | gcgccgtctcgctcgaatggaatctcctgctactaaatta | 12 |
| pUPD2-Dom-CsCCD2-L-R1 | gcgccgtctcgttgtctctgcctcctcctta | 13 |
| pUPD2-Dom-CsCCD2-L-F2 | gcgccgtctcgacaagtaagaagaagcccaaac | 14 |
| pUPD2-Dom-CsCCD2-L-R2 | gcgccgtctcgctcaaagctcatgtctctgcttggtgct | 15 |
| pUPD2-Dom-UGT709-F | gcgccgtctcgctcgaatggctgagaaagaagcaaatac | 16 |
| pUPD2-Dom-UGT709-R | gcgccgtctcgctcaaagctcaggtccgaagaaaatttgg | 17 |
| pUPD2-Dom-pPAT-F | gcgccgtctcgctcgggagaatttgtcaaatcaggctcaaaga | 35 |
| pUPD2-Dom-pPAT-R | gcgccgtctcgctcacattctttgcaaatgttcaaagtgttttta | 36 |

The products were cloned in the level 0 vector pUPD2, obtained from (https://gbcloning.upv.es, Valencia, Spain). The resulting plasmids pUPD2-CsCCD2L (SEQ ID NO: 18), pUPD2-CsUGT2 (SEQ ID NO: 19), and pUPD-CsUGT709G1 (SEQ ID NO: 20) were then used to construct four recombinant binary plasmids, according to the Goldenbraid modular cloning system, as follows:
- O1 (SEQ ID NO: 21) = pDGB3α1[p35S:UGT2:T35S-p35S:UGT709G1:T35S-pE8:CCD2L:T35S-pNos:Hyg:T35S] **(****Figure 1A****),**
- O2 (SEQ ID NO: 22) = pDGB3α1[p2A11:UGT2:T35S-p35S:UGT709G1:T35S-pE8:CCD2L:T35S-pNos:Hyg:T35S], **(****Figure 1B**)
- O3 (SEQ ID NO: 23) = pDGB3α1[p35S:UGT2:T35S-p35S:UGT709G1:T35S-p35S:CCD2L:T35S-pNos:Hyg:T35S], **(****Figure 1C****),** and
- O4 (SEQ ID NO: 24) = pDGB3α1[pE8:UGT2:T35S-p35S:UGT709G1:T35S-p2A11:CCD2L:T35S-pNos:Hyg:T35S] **(****Figure 1D****).**
- O6 (SEQ ID NO: 34) = pDGB3α2[pPatGT2T35S-pPatUGT709T35S-pPatCCD2LT35S-pNos-Hyg-T35S] **(****Figure 1E**)

### Tomato transformations

Binary plasmids (O1 to O4, SEQ ID NO: 21 to 24, respectively) were transferred to *Agrobacterium tumefaciens* LBA4404 strain by electroporation and then used for tomato stable transformation into *S*. *lycopersicum* (var. Moneymaker), as described previously (Ellul, P. et al. Theor Appl Genet. 2003, 106, 231-238). Transgenic plants were selected for their ability to develop roots on hygromycin selection media and the genomic DNA was further verified using PCR to check for the presence of the corresponding genes (see below). All *in vitro* steps were carried out in a long-day growth chamber (16 h light/8 h dark, 24°C, 60-70% humidity, 250 µmol/m²/s). Positive transgenic tomato plants were transferred to soil and grown in a greenhouse with a 14/10 h day/night photoperiod at 22 °C.

### Potato transformations

Binary plasmid O6 (SEQ ID NO: 34) was transferred to the *Agrobacterium tumefaciens* LBA4404 strain by electroporation and used for stable potato cv. Desirée and cv. OIH15Wt transformation into *S*. *tuberosum,* as described previously (Tavazza et al. Plant Science 59(2): 175-181. 1989). Transgenic plants were selected for their ability to develop roots on hygromycin selection media and the genomic DNA was further verified using PCR to check for the presence of the corresponding genes (see below). All *in vitro* steps were carried out in a long-day growth chamber (16 h light/8 h dark, 24°C, 60-70% humidity, 250 µmol/m²/s). Positive transgenic potato plants were transferred to soil and grown in a greenhouse with a 14/10 h day/night photoperiod at 22 °C.

### Apocarotenoid and carotenoid analyses

Metabolite extraction and analysis differed depending on the nature of the metabolites. Polar and non-polar metabolites were extracted from 50 mg and 5 mg of lyophilized fruit/tuber tissues, respectively. For the polar metabolite analyses (crocins and picrocrocins), the tissue was extracted in cold 75% methanol. The soluble fractions were analyzed using high performance liquid chromatography-diode array detector-high resolution mass spectrometry (HPLC-DAD-HRMS) and HPLC-DAD as previously described (Diretto, G. et al. New Phytol. 2019; 16079). The apolar fractions (crocetin, HTCC (4-hydroxy-2,6,6-trimethyl-1-cyclohexene-1-carboxaldehyde) and carotenoids) were extracted with 1:2 cold extraction solvents (50:50 methanol:CHCl₃), and analyzed by HPLC-DAD-HRMS and HPLC-DAD as previously described (Diretto, G. et al. New Phytol. 2019; 16079).

Metabolites were identified using co-migration with standards, by matching the UV spectrum of each peak against that of a standard when available, on the basis of literature data, and m/z accurate masses, as reported in the Pubchem database (http://pubchem.ncbi.nlm.nih.gov/) for monoisotopic mass identification or using the Metabolomics Fiehn Lab Mass Spectrometry Adduct Calculator (http://fiehnlab.ucdavis.edu/staff/kind/Metabolomics/MS-Adduct-Calculator/) in the case of adduct detection. Pigments were quantified by integrating the peak areas that were converted to concentrations by comparison with the standards and as reported previously (Martí, M. et al. Metabolic Engineering. 2020, 61, 238-250).

### Volatile analyses

Volatile compounds were captured by means of headspace solid phase microextraction (HS-SPME) and analyzed by gas chromatography coupled to mass spectrometry (GC/MS). Tomato fruits were collected at the red ripe stage, and sections of the pericarp were excised and immediately frozen in liquid nitrogen. For metabolite extraction, frozen tomato pericarp sections from 3-5 fruits representing the transformation events were ground in a cryogenic mill and stored at -80°C until analysis, while three weeks after the plant's foliage has died back mature potatoes were harvest and frozen tuber potato sections from 3-5 potatoes were ground in a cryogenic mill. Analyses of the volatile compounds was performed similarly to a previously described process (Raines, C., et al. Journal of experimental botany. 2017, 68, 347-349). Roughly, 500 mg of the resulting powder was introduced into a 15 mL glass vial and incubated at 37°C for 10 min in a water bath. Then, 500 µL of an EDTA 100 mM, pH 7.5 solution and 1.1 g of CaCl₂. 2H₂O were added, mixed gently, and sonicated for 5 min. After this, 1 mL of the resulting paste was transferred to a 10 mL screw cap headspace vial with silicon/PTFE septum and analyzed within 12 hours. Volatile compounds were extracted from the headspace by means of a 65 µm PDMS/DVB solid phase microextraction fiber (SUPELCO). Volatile extraction was performed automatically by means of a CombiPAL autosampler (CTC Analytics). Vials were first incubated at 50°C for 10 min with agitation at 500 rpm. Then, the fiber was exposed to the headspace of the vial for 20 min under the same temperature and agitation conditions. Desorption was performed at 250°C for 1 min using the splitless mode in the injection port of a 6890N gas chromatograph (Agilent Technologies). After desorption, the fiber was cleaned in an SPME fiber conditioning station (CTC Analytics) at 250°C for 5 min, under a helium flow. Chromatography was performed on a DB-5ms (60 m, 0.25 mm, 1.00 µm) capillary column (J & W) with helium as the carrier gas at a constant flow of 1.2 mL/min. The GC interface and MS source temperatures were 260°C and 230°C, respectively. Oven programming conditions were 40°C for 2 min, 5°C/min ramp until 250°C, and a final hold at 250°C for 5 min. Data was recorded in a 5975B mass spectrometer (Agilent Technologies) in the 35-250 m/z range at 6.2 scans/s, with electronic impact ionization at 70 eV. Chromatograms were processed using the Enhanced ChemStation E.02.02 software (Agilent Technologies).

Identification of the compounds was performed by comparing both the retention times and the mass spectrum data with those of the pure standards. When standards were not available, a tentative identification was performed based on mass spectrum similarities in the NIST05 Mass Spectral Library.

For quantitation, one specific ion was selected for each compound, and the corresponding peak from the extracted ion chromatogram was integrated. The criteria for ion selection were having the highest signal-to-noise ratio and being specific enough to provide good peak integration in the specific region of the chromatogram. An admixture reference sample was prepared for each season by mixing thoroughly equal amounts of each sample. A 500 mg aliquot of the admixture was analyzed regularly (one admixture sample for every six to seven samples) and processed as a regular sample as part of the injection series. This admixture contained all the compounds identified in the individual samples at an intermediate concentration, and was used as a reference to normalize for temporal variation and fiber aging. Finally, the normalized results (corrected for temporal variation and fiber aging) for a sample were expressed as the ratio of the abundance of each compound in a specific sample to those present in the reference admixture.

### Un-target metabolomic analyses by High-Resolution Mass Spectrometry (LC-HRMS)

Two biological replicates and two technical replicates were processed and analyzed independently. For polar metabolites 10 mg of freeze-dried fruit powder was extracted with 0.75 mL cold 75% (v/v) methanol with 0.5 mg/L formononetin as the internal standard, and for non-polar analyses 3 mg of powder was extracted with 0.25 mL cold 100% (v/v) methanol, 1 mL of CHCl₃ with 10 mg/L α-tocopherol acetate as the internal standard, and 0.25 mL of the 50 mM Tris buffer (pH 7.5, with 1 M NaCl). Liquid chromatography coupled to high-resolution mass spectrometry (LC-HRMS) conditions were as previously reported (Dono, G. et al. Metabolites. 2020, 10).

### mRNA expression studies

RNA was extracted from 100 mg of fresh fruits/tubers with the Direct-Zol RNA MicroPrep kit (Zymo Research) following the manufacturer's instructions. Synthesis of the cDNA from each transgenic line and wild type was performed using oligo-dT and the Ready-To-Go You-Prime First-Strand Beads (Ge Healthcare), following the manufacturer's instructions. qPCR was then carried out using the primers listed in **Table 2,** and the GoTaq^{®} qPCR Master Mix (Promega, Madison, WI, USA), following the manufacturer's instructions. The constitutively expressed *Actin-2* gene transcript was used as a reference gene. The method was as follows: initial denaturation at 94 °C for 5 min; 30 cycles of denaturation at 94 °C for 20 s, annealing at 60 °C for 20 s, and extension at 72 °C for 20 s; and a final extension at 72 °C for 5 min. Assays were done in a StepOne^{™} Thermal Cycler (Applied Biosystems,) and analyzed using the StepOne software v2.0 (Applied Biosystems).

**Table 2. Oligonucleotides used for expression analyses.**

| **Gene name** | **Oligonucleotide forward 5'-3'** | **Oligonucleotide reverse 5'-3'** |
|---|---|---|
| *CsUGT2* | tcgagcctagtgatctgccgt (SEQ ID NO: 25) | tcgagccagtccaagtaggga (SEQ ID NO: 26) |
| *UGT709G1* | actcacctccacgtccttccag (SEQ ID NO: 27) | cttgatcagccacgacacaag (SEQ ID NO: 28) |
| *CsCCD2L* | acatgtcgccttgagagtcc (SEQ ID NO: 29) | tcagatttgatgccaggttg (SEQ ID NO: 30) |
| *Actin-2 tomato* | cattgtgctcagtggtggttc (SEQ ID NO: 31) | tctgctggaaggtgctaagtg (SEQ ID NO: 32) |
| *Actin-potato* | gcttcccgatggtcaagtca (SEQ ID NO:37) | ggattccagctgcttccattc (SEQ ID NO: 38) |

### 2,2-Diphenyl-1-picrylhydrazyl (DPPH) radical scavenging activity

Free radical scavenging (FRS) activity was analyzed using fruit/tuber samples (50 mg lyophilized fruits/tubers) that were extracted with acetone and mixed with 0.2 mM methanolic DPPH radical solution (0.5 mL). Equal volumes (1 mL) of tomato fruit or tuber extracts and DPPH solution (0.2 mM in ethanol) were mixed and kept in the dark at room temperature for 30 min. The absorbance of the solution was measured at 517 nm. The FRS was calculated by %= (A₀-A₁/A₀) × 100.

### Statistics

For the study of the plant material, three to five biological replicates with three technical replicates per biological replicate were analyzed for every experiment. The obtained data were statistically analyzed with one-way analysis of variance and Duncan's test of significance using the SPSS software. Levels of significance were determined by t-test.

### Example 1. Generation of plasmids for ectopic expression of the saffron apocarotenoid pathway in tomato transgenic lines.

Initially, three different promoters were selected and evaluated for saffron gene expressions in tomato (*S. lycopersicum* cv. Moneymaker (MM)). The pE8 (SEQ ID NO: 7) and p2A11 (SEQ ID NO: 8) are fruit specific promoters from tomato, whose activity could result in high levels of accumulation of the desired metabolites without yield penalties, because fruit set, development, and ripening are mostly completed at the time of their activation. These promoters were used for *CsCCD2L* gene expression in constructs O1 (SEQ ID NO: 21) (pE8), O2 (SEQ ID NO: 22) (pE8), and O4 (SEQ ID NO: 24) (p2A11), and for *CsUGT2* expression in construct O2 (SEQ ID NO: 22) (p2A11) and O4 (SEQ ID NO: 24) (pE8). The constitutive CaMV 35S promoter (SEQ ID NO: 9) was used to drive the expression of all the genes in construct O3 (SEQ ID NO: 23), of *CsUGT2* and *UGT709G1* in construct O1 (SEQ ID NO: 21), of *UGT709G1* in construct O2 (SEQ ID NO: 22), and of *UGT709G1* in construct O4 (SEQ ID NO: 24). Four different binary plasmids were created, O1 (SEQ ID NO: 21), O2 (SEQ ID NO: 22), O3 (SEQ ID NO: 23) and O4 (SEQ ID NO: 24) and used for the tomato transformations **(****Figure 1A-D****).**

The tomato variety MM was selected for Agrobacterium-mediated transformation as it is widely used in tomato genetic studies. Tomato transgenic lines were obtained for all constructs: O1 (20 lines), O2 (3 lines), O3 (5 lines), and O4 (12 lines). Ten lines able to produce fruits, were selected and the expression levels of the introduced transgenes were confirmed by qRT-PCR analysis **(****Figure 2A****).** Seeds were collected from fruits of the primary transgenic lines. Primary transformants carrying the construct O3 failed to produce any viable seeds **(****Figure 3B****),** but these fruits showed a striking orange color, and differences in the coloration of its flower parts were observed **(****Figure 3A-D****).** All the other primary transgenic lines presented flowers and fruits with no obvious phenotypic differences compared to the wild-type (WT). Interestingly, these latter lines produced fruit with viable seeds **(****Figure 4****),** which showed differences in fruit color appearance in the next generations.

In addition, all the transgenic lines showed higher antioxidant activity than the untransformed WT plants used as the control, based on its radical scavenging activity **(****Figure 5****).** Compared with the fruit of wild-type MM, the antioxidant capacity of the transgenic lines was about 3 to 7-fold higher. In addition, **Table 3** shows the profile of individual phenolic acids and flavonoids in tomato and tomato by-product extracts determined by HPLC-DAD-HRMS. Results are presented as the ratio of transgenic lines vs WT. * Indicates significant statistical differences with p-value < 0.05.

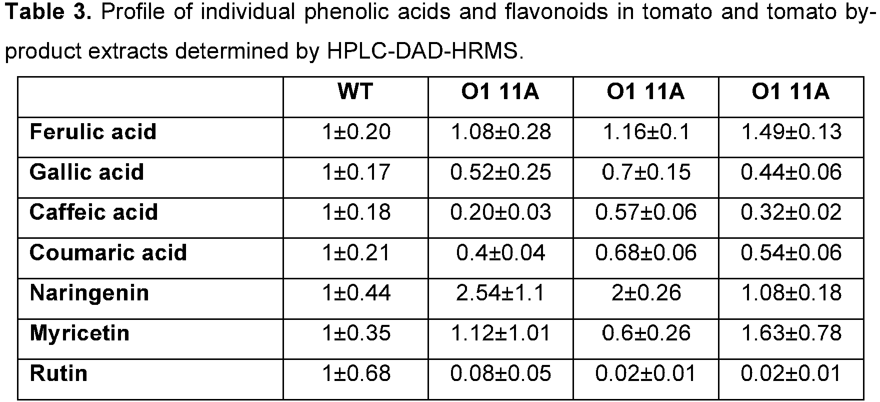

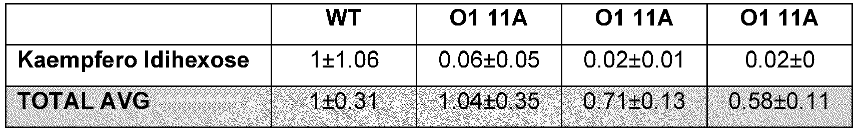

### Example 2. Apocarotenoid evaluation of fruits from tomato transgenic plants.

Fruits of transgenic lines O1, O2, and O4, were evaluated for the presence of saffron apocarotenoids **(Tables 4, 5 and 6).** The analyzed lines showed the presence of all the apocarotenoids that are characteristic of saffron at variable levels, including crocin 3 [(crocetin-(β-D-gentiobiosyl)-(β-D-glucosyl)-ester)], which was preferentially accumulated **(Table 4).** Fruits were dissected and the different tissues analysed for crocins, indicating that crocins preferentially accumulated in the placenta tissue, followed by in the pericarp **(Table 5).** The levels HTCC and safranal, were also analysed in these fruits **(Table 6).** All three were detected in all the lines analysed, and were, as expected, absent in the fruits of the WT plants.

**Table 4. Crocetin, picrocrocin and crocin contents in the WT and transgenic lines (mg/g DW).**

| | **WT** | **O1 11A** | **O2 B** | **O3 2B** | **04 12** |
|---|---|---|---|---|---|
| **trans-crocin-4** | 0.00 | 1.82±0.82 | 1.81±0.79 | 0.93±0.37 | 0.71±0.22 |
| **trans-crocin-3** | 0.00 | 6.53±0.68 | 9.07±1.75 | 11.58±1.37 | 5.56±2.09 |
| **cis-crocin-3** | 0.00 | 0.05±0.03 | 0.00 | 0.00 | 0.00 |
| **trans-crocin-2'** | 0.00 | 0.12±0.04 | 0.52±0.18 | 0.11±0.05 | 0.00 |
| **cis-crocin-2'** | 0.00 | 0.04±0.02 | 0.27±0.05 | 0.06±0.03 | 0.00 |
| **trans-crocin-2** | 0.00 | 3.10±0.7 | 1.16±0.44 | 1.94±0.38 | 1.41±0.18 |
| **trans-crocin-1** | 0.00 | 0.09±0.06 | 0.16±0.09 | 0.09±0.03 | 0.00 |
| **cis-crocin-1** | 0.00 | 0.14±0.1 | 0.05±0.02 | 0.03±0.01 | 0.16±0.07 |
| **trans-crocetin** | 0.00 | 0.01±0.01 | 0.02±0 | 0.01±0.0 | 0.00 |
| **picocrocin** | 0.00 | 1.93±0.6 | 1.43±0.56 | 2.65±0.01 | 2.68±0.51 |

**Table 5. Percentage of the crocins that accumulated in the different parts of the tomato fruit.**

| | **WT** | **O1 11A** | **O2 B** | **O3 2B** | **04 12** |
|---|---|---|---|---|---|
| **Exocarp** | 0 | 17.81 | 3.46 | 6.07 | 10.62 |
| **Pericarp** | 0 | 26.78 | 36.22 | 46.4 | 21.33 |
| **Placenta** | 0 | 55.4 | 60.32 | 47.52 | 68.04 |

**Table 6. HTCC and safranal contents in the WT and transgenic lines (fold IS).**

| | **WT** | **O1 11A** | **O2 B** | **O3 2B** | **04 12** |
|---|---|---|---|---|---|
| **safranal** | 0 | 0.47±0.02 | 0.38±0.15 | 0.79±0.13 | 1.83±0.17 |
| **HTCC** | 0 | 5.51±0.74 | 5.18±1.13 | 5.96±0.09 | 7.33±0.29 |

The levels of ABA and its storage form (7 ABA-glucose ester) were also analysed in these lines. Overall, the levels of these metabolites were reduced in the transgenic fruits **(****Figure 6****),** suggesting that zeaxanthin conversion to crocetin dialdehyde impairs ABA biosynthesis, but without affecting the development or viability of the seeds with the exception of the O3 lines in which *CsCCD2L* under the 35S promoter, was unable to produce seeds, which suggested that constitutive *CsCCD2L* expression was accompanied by detrimental effects during seed development. **(****Figure 4****).**

### Example 3. Levels of carotenoids and related compounds in WT and transgenic tomato fruits.

Transgenic and control (WT) fruits were subjected to carotenoid extraction and HPLC-APCI-HRMS analyses to determine their carotenoid profiles **(Table 7).** In general, the carotenoid content levels in all the transgenic fruits were remarkably reduced compared to that in the WT. The most striking differences were related to lycopene, β-carotene, and lutein content levels. Indeed, lutein and α-carotene levels were reduced or undetectable in the transgenic lines **(Table 7).**

**Table 7. Carotenoid content in the tomato fruits from the WT and transgenic lines. Results are presented as the ratio of transgenic lines vs WT.**

| | **WT** | **O1 11A** | **O2 B** | **O3 B** | **04 12** |
|---|---|---|---|---|---|
| **phytoene** | 1±0.22 | 0.65±0.17 | 0.52±0.16 | 0.54±0.08 | 0.84±0.12 |
| **α-carotene** | 1±0.2 | nd | nd | nd | nd |
| **β-carotene** | 1±0.24 | 0.03±0.02 | 0.02±0.01 | 0.03±0.005 | 0.01±0.003 |
| **lutein** | 1±0.12 | nd | nd | 0.18±0.04 | 0.02±0 |
| **lycopene** | 1±0.11 | 0.12±0.02 | 0.05±0.01 | 0.06±0.02 | 0.06±0.01 |
| **Total carotenoids** | 1±0.13 | 0.19±0.05 | 0.11±0.03 | 0.14±0.02 | 0.18±0.03 |

### Example 4. Evaluation of the crocins in T2 fruits.

Fruits from T2 plants were analysed for their total crocins and picrocrocin content **(****Figure 7****).** The fruits showed no colour changes in their peels. However, when cut in half, an orange coloration could be observed in their internal part **(****Figure 8****).** An evaluation of the total crocins content in the complete fruit and in the tomato juice serum of these selected lines was performed **(****Figure 7****).**

Tomatoes were crushed and the serum juice obtained after centrifugation **(****Figure 7****).** This serum from the transgenic lines showed a yellow to orange colour in comparison to the almost colourless extract from the WT fruits **(****Figure 7****).** Crocins were detected at 440 nm in all the yellow-orange serum juices, but not in the control tomato serum juice **(****Figure 7****).** No differences were found in total phenolics and flavonoids between the transgenic and WT serum juices **(****Figure 8****).** Fruits with up to 12.48 mg/g of dry weight of crocins were obtained from line O1 11 **(Table 8).** This line showed the highest levels of crocins in the serum juice **(Table 8).**

**Table 8. Content of saffron apocarotenoids in fruits and serum juice of WT and transgenic lines.**

| **Plants** | **Fruit** | | **Juice** | |
|---|---|---|---|---|
| | **crocins (mg/g DW)** | **picrocrocin (mg/g DW)** | **crocins (g/L)** | **picrocrocin (g/L)** |
| **O2 B** | 5.32 ± 0.1 | 1.49 ± 0.22 | 0.066 ± 0.03 | 0.0153 ± 0.00 |
| **O4 12** | 8.77 ± 0.23 | 2.92 ± 0.09 | 0.106 ± 0.00 | 0.0274 ± 0.00 |
| **O1 11A** | 12.48 ± 0.18 | 2.42 ± 0.06 | 0.153 ± 0.02 | 0.0267 ± 0.00 |
| **WT** | nd | nd | nd | nd |

An additional experiment was performed to compare the pigmentation power of the dry tomatoes with that of saffron threads in a simulated cooking process. For this experiment, two procedures were followed, in the first, dry transgenic tomato (0.622 g), the same quantity of tomato WT, and the threads from one flower of saffron (0.002 g), were immersed each in 50 mL of water at 90 °C; in the second procedure, 0.02 g of powdered dry transgenic tomato, WT tomato, and saffron were combined with 5 ml of water. The diffusion of the crocins in the water was analysed by HPLC-DAD at 10, 30, and 60 min after the introduction of the samples in the solution **(****Figure 9****).** The sliced dry tomatoes showed a rapid release of crocins compared to the saffron stigmas, however after 60 min the colouring power of the saffron was much higher. When the powder of the transgenic dry tomatoes was used, the release of the crocins and the colouring power was greater with the cut tomatoes.

The results showed the clear colouring power of the engineered tomato, although different crocin kinetics of accumulation in the solution were observed, depending on the material used, and the method of sample preparation. In the tomato fruit, the uniform mixture of the dry powder was more efficient than the sliced one, which clearly affected the diffusion of the crocins in the solution **(****Figure 9****).** Such differences could be due to the presence of the tomato peel, preventing the spread of crocins from the inside, and provide interesting hints about the potential industrial exploitation of these high-crocins tomatoes. The accumulation the crocins in water is shown in **Table 9.**

**Table 9. Accumulation the crocins (µg/mg DW) in water.**

| | **Time (min.)** | | |
|---|---|---|---|
| **Sample** | **10** | **30** | **60** |
| **O1 11** | 0,16 | 0,36 | 0,527 |
| **O1 11 powder** | 2,8 | 3,14 | 3,41 |
| **Saffron** | 49,5 | 264,75 | 425 |
| **Saffron powder** | 75,42 | 278,79 | 395,5 |

Thus, the data shown herein, revealed picrocrocin and crocetin-(β-D-gentiobiosyl)-(β-D-glucosyl)-ester, as the predominant crocin molecules, as well as safranal at the expense of the usual tomato carotenoids. The results show the highest crocin content ever obtained by metabolic engineering in heterologous systems, and provides a new platform to produce economically competitive saffron apocarotenoids.

In summary, the data provided herein demonstrate the potential and feasibility of using tomatoes as biotechnological tools for the simultaneous production of pro-nutritional lipophilic and rare high-valuable hydrophilic apocarotenoid compounds that are beneficial for both industry and human health. The results show the highest crocin content ever obtained by metabolic engineering in heterologous systems, and provides a new platform to produce economically competitive saffron apocarotenoids.

### Example 5. Study of the effect of crocins and picrococin produced by transgenic tomato in the treatment of Alzheimer using C. elegans as animal model.

The anti-oxidant and anti-inflammatory properties of saffron has been demonstrated in cardiovascular, digestive, and ocular diseases, and in leukemia. Furthermore, the effect of crocin-1 in Alzheimer disease has been also demonstrated in a mouse hippocampal neuronal cell line (HT22), and in Alzheimer disease animal models such as mice and rat models. Moreover, in a human trial with patients suffering from mild to moderate Alzheimer, patients that received 15 mg of saffron twice per day for 16 weeks showed a better cognitive function than patients who received the placebo (Akhondzadeh et al., J. Clin. Pharm. Ther., 2010; 35, 581-588).

The aim of the example was to test the effect of crocins and picrocrocins produced in tomato in a *C*. *elegans* Alzheimer model.

### Materials and methods

### Plant material

Wild-type and transgenic tomato obtained from the transgenic plant of the present invention (*Solanum lycopersicum* cv. Money Maker) were independently crushed in a blender, the obtained juice was centrifuged at 9,000g for 15 minutes. The liquid phase was collected and lyophilized for 2 days. The lyophilized of *wild-type* and transgenic extracts were weighted, resuspended in distilled water, and diluted at six different concentrations (0.04, 0.4, 0.4, 4, 10, and 20 mg of extract/ml). The transgenic extract contained 2.54 mg of crocins per gram of extract, the crocin titer in each dilution is listed in **Table 10** below.

**Table 10. Crocin content in the evaluated extract dilutions. The initial concentration of crocins in the transgenic tomato extracts was 2.54 mg/g.**

| **Extract dilution (mg/ml)** | **Crocins (µg/ml)** |
|---|---|
| 0.04 | 0.1 |
| 0.2 | 0.5 |
| 0.4 | 1 |
| 4 | 10 |
| 10 | 25 |
| 20 | 50 |

### C. elegans strain for Alzheimer disease

The transgenic *C*. *elegans* CL4176 strain was used to carry out the experiment. This strain expresses the human amyloid β-peptide 1-42 (Aβ₁₋₄₂) peptide at 25°C. The worms were synchronized at 16°C in NGM media (negative control) or or the different dilutions of non-transgenic or transgenic tomato extracts **(Table 10).** The temperature was increased to 25°C to activate the Aβ₁₋₄₂ expression, and the percentage of paralyzed worms was scored at different times (20, 24, 26, 28, 30, and 32 hours). A control without induction was included (NGM without induction). A total of two replicas were performed for each extract and dosage.

### Results

Both extracts from *wild-type* and transgenic tomato showed a protective effect against worm paralysis in all analyzed concentrations comparing with the negative control at the different analysis times. The most effective concentration in both extracts was 4 mg/ml, corresponding to 10 µg/ml of crocins in transgenic tomato extracts. The same effect was observed at higher concentrations (10 and 20 mg/ml of extract). After 32 hours of paralysis induction through Aβ1-42 expression, the wild-type extract at 4 mg/ml showed a significant lower effect comparing with the transgenic extract at 4 mg/ml **(****Figure 10****).**

Accordingly, the results show that the transgenic tomato extract showed a significant lower paralysis of *C*. *elegans* CL4176 with induced Aβ₁₋₄₂ expression than the control tomato extract. The effect of the transgenic tomato extract was the same at the three highest crocin concentrations tested (10, 25, and 50 µg/ml). Therefore, the crocins produced by the transgenic tomatoes are useful for the treatment of Alzheimer disease.

### Example 6. Generation of plasmids for ectopic expression of the saffron apocarotenoid pathway in potato transgenic lines.

In order to avoid possible pleiotropic effects on potato plant development by using a constitutive promoter to over-express the three genes, UGT2, UGT709G1 and CCD2L of the invention, involved in the saffron apocarotenoid pathway, the construct was engineered containing the tuber-specific patatin promoter pPat (SEQ ID NO: 33) was selected and a binary construct was assembled using the Goldenbraid strategy, carrying the *CsCCD2L, UGT74AD1* and *UGT709G1* coding sequences driven by the patatin promoter pPat (SEQ ID NO: 33). Thus, the binary construct O6 (SEQ ID NO: XX) was created and used for the potato transformation **(****Figure 1E****).**

*A. tumefaciens*-mediated transformation was then used to introduce the transgenes into *S*. *tuberosum* cv. Désirée and cv OIH15WT. Approximately 10 independents transgenic lines of each variety were generated. The expression levels of the introduced transgenes were confirmed by qRT-PCR analysis **(****Figure 2B****).** Based on visual assessment of tuber color, 2 lines were selected for detailed analysis **(****Figure 3E****).**

In addition, all the transgenic lines showed higher antioxidant activity than the untransformed WT plants used as the control, based on its radical scavenging activity (data not shown). Compared with the WTs, the antioxidant capacity of the transgenic lines was about 2 to 7-fold higher.

### Example 7. Apocarotenoid evaluation of tubers from potato transgenic plants.

All potato lines, WTs and transgenics showed the presence of all the apocarotenoids characteristic of saffron, albeit at variable levels **(Tables 11 and 12).** The analyzed lines showed the presence of all the apocarotenoids that are characteristic of saffron at variable levels, including crocin 3 [(crocetin-(β-D-gentiobiosyl)-(β-D-glucosyl)-ester)] **(Table 11).** The levels HTCC and safranal, were also analysed in these lines **(Table 12).** All three were detected in all the lines analysed, and were, as expected, absent in the tubers of the WTs plants.

**Table 11. Crocetin, picrocrocin and crocin contents in the WTs and transgenic lines (µg/g).**

| | **WTs** | | **Transgenic potato plants** | |
|---|---|---|---|---|
| | **Désireé** | **OIH15WT** | **O6 5C** | **OIH15WT-O6** |
| picrocrocin (Total) | 0.00 | 0.00 | 53.941±4.721 | 168.518±15.363 |
| trans crocetin | 0.00 | 0.00 | 38.44±2.097 | 457.61±25.67 |
| cis crocetin | 0.00 | 0.00 | 0.00 | 0.00 |
| trans crocin 1 | 0.00 | 0.00 | 5.41±4.36 | 37.94±4.03 |
| cis crocin 1 | 0.00 | 0.00 | 0.00 | 18.4±2.34 |
| trans crocin 2 | 0.00 | 0.00 | 51.08±3.27 | 120.99±14.32 |
| trans crocin 2' | 0.00 | 0.00 | 110.94±13.56 | 949.71±156.09 |
| cis crocin 2+2' | 0.00 | 0.00 | 6.91±1.34 | 62.00±7.67 |
| trans crocin 3 | 0.00 | 0.00 | 47.91±6.34 | 3182±543.09 |
| cis crocin 3 | 0.00 | 0.00 | 17.49±4.56 | 768.31±34.42 |
| trans crocin 4 | 0.00 | 0.00 | 35.89±8.71 | 312.21±54.21 |

**Table 12. HTCC and safranal contents in the WT and transgenic potato lines. Results are presented as the ratio of transgenic lines vs WT.**

| | **WTs** | | **Transgenic potato plants** | |
|---|---|---|---|---|
| | **Desirée** | **OIH15 WT** | **O6 5C** | **OIH15 WT-O6** |
| **HTTC** | 0.00 | 0.00 | 5.885±0.287 | 38.178±3.24 |
| **Safranal** | 0.00 | 0.00 | 0.01±0.002 | 0.04±0.001 |

### Example 8. Levels of carotenoids and related compounds in WTs and transgenic potato tubers.

Transgenic and control (WT) potato tubers were subjected to carotenoid extraction and HPLC-APCI-HRMS analyses to determine their carotenoid profiles **(Table 13).** In general, the carotenoid content levels in all the transgenic fruits were remarkably reduced compared to that in the WT.

**Table 13. Carotenoid content in tubers from the WTs and transgenic lines. Results are presented as the ratio of transgenic lines vs WT.**

| | **WTs** | | **Transgenic potato plants** | |
|---|---|---|---|---|
| | **Desireé** | **O1H15WT** | **O6 5C** | **O1 H15 WT 06** |
| **Neoxanthin** | 1±0.005 | 1±0.006 | 0.457±0.003 | 0.7291±0.009 |
| **Violaxanthin** | 1±0.013 | 1±0.019 | 2.355±0.023 | 0.2434±0.004 |
| **Luteoxanthin** | 1±0.001 | Nd | 0.61±0.001 | Nd |
| **Antheraxanthin** | 1±0.015 | 1±0.057 | 0.054±0.002 | 0.0496±0.018 |
| **Phytoene** | Nd | 1±0.092 | Nd | 0.2346±0.068 |
| **Phytofluene** | Nd | 1±0.021 | Nd | 0.78±0.030 |
| **Zeaxanthin** | 1±0.011 | 1±0.595 | nd | 0.019±0.034 |

## Claims

1. A DNA construct comprising nucleotide sequences encoding for a combination of three proteins for apocarotenoids compounds biosynthesis, preferably crocins, crocetins, picrocrocin, HTTC and safranal, wherein the proteins are selected from UGT2, UGT709G1 and CCD2L, or a functionally equivalent sequence thereof, and wherein these proteins preferably belong to genus *Crocus,* preferably from *C*. *sativus,*
wherein the amino acid sequences for UGT2, UGT709G1 and CCD2L, or a functionally equivalent sequence thereof, are selected from an amino acid sequence at least 90%, 91%, 92%, 93%, 94%,95%, 96%, 97%, 98% or 99% identical to the sequences selected from the list consisting of SEQ ID NO: 4, SEQ ID NO: 6 and SEQ ID NO: 2 respectively,
wherein each nucleotide sequence is operably linked to a promoter selected from a fruit-specific promoter, tuber-specific promoter and/or a constitutive promoter, and
wherein the nucleotide sequence encoding for the CCD2L is operably linked to a fruit-specific promoter or a tuber-specific promoter.

2. The DNA construct according to claim 1, wherein the UGT2 protein is encoded by a nucleotide sequence having at least 90%, 91%, 92%, 93%, 94%,95%, 96%, 97%, 98% or 99% identity to SEQ ID NO: 3, the UGT709G1 protein is encoded by the nucleotide sequence having at least 90%, 91%, 92%, 93%, 94%,95%, 96%, 97%, 98% or 99% identity to SEQ ID NO:5, and the CCD2L protein is encoded by the nucleotide sequence having at least 90%, 91%, 92%, 93%, 94%,95%, 96%, 97%, 98% or 99% identity to SEQ ID NO: 1.

3. The DNA construct according to any one of claims 1 to 2, wherein the fruit-specific promoter is selected from the list consisting of: tomato E8 promoter (SEQ ID NO: 7) and p2A11 promoter (SEQ ID NO: 8), the constitutive promoter is the Cauliflower Mosaic Virus 35S promoter (SEQ ID NO: 9) and the tuber specific promoter is the patatin promoter pPat (SEQ ID NO: 33).

4. The DNA construct according to any one of claims 1 to 3, wherein the constructs are selected from the list consisting of SEQ ID NO: 21; SEQ ID NO: 22, SEQ ID NO: 23, SEQ ID NO: 24 and SEQ ID NO: 34).

5. A method for increasing the levels of apocarotenoids compounds, preferably crocins, picrocrocins, HTTC and safranal in a Solanaceous plant, wherein the method comprises introducing and expressing the DNA construct according to claims 1 to 4 into the Solanaceous plant.

6. A method for producing a transgenic Solanaceous plant having increased levels of apocarotenoids compounds, preferably crocins, picrocrocins, HTTC and safranal, compared to the corresponding wild-type Solanaceous plant, wherein the method comprises introducing and expressing the DNA construct according to claims 1-4 into the Solanaceous plant.

7. The method according to any one of claims 5 or 6, wherein the Solanaceous plant is selected from the species *Solanum tuberosum* (potato plant) and *Solanum lycopersicum* (tomato plant).

8. A transgenic Solanaceous plant, which comprises in its genome the DNA construct according to any one of claims 1 to 4, wherein the Solanaceous plant accumulates increased levels of apocarotenoids compounds, preferably crocins, picrocrocins, HTTC and safranal compared to the corresponding wild-type Solanaceous plant.

9. The transgenic Solanaceous plant according to claim 8, wherein it is selected from the list consisting of *Solanum tuberosum* (potato plant) and *Solanum lycopersicum* (tomato plant).

10. The transgenic Solanaceous plant according to any one of claims 8 to 9, wherein the increased levels of apocarotenoids compounds, preferably crocins, picrocrocins, HTTC and safranal are increased in harvestable parts of the plant, preferably in tubers, fruit tissues or seeds.

11. Harvestable parts of the Solanaceous plant according to any one of claims 8 to 10 selected from tubers, seeds and fruits. having increased levels of apocarotenoids compounds.

12. A food product comprising the harvestable part of the Solanaceous plants according to claim 11, wherein the food product has enhanced apocarotenoids compounds, preferably crocins, picrocrocins, HTTC and safranal levels.

13. The food product according to claim 12, wherein the food product is selected from the list consisting of a tomato, a tomato-based product, a potato and a potato-based product, preferably wherein the tomato-based product is selected from the list consisting of ketchup, a tomato-based sauce, a pizza sauce, tomato soup or tomato juice and wherein, preferably, the potato-based product is selected from the list consisting of: a potato flakes, potato chips, or potato flour.

14. A pharmaceutical or nutraceutical composition comprising the harvestable part of the Solanaceous plants according to claim 11, or the food product according any one of claims 12 to 13, a fragment or an extract thereof, and a pharmaceutical or nutraceutical acceptable carrier.

15. The harvestable part of the Solanaceous plants according to claim 11, the food product according to any one of claims 12 to 13, the nutraceutical composition or the pharmaceutical composition according to claim 14, for use as medicament, preferably for use in the prevention and/or treatment of neurodegenerative diseases preferably selected from the list consisting of: Alzheimer disease and Parkinson disease.

## Patentansprüche

1. Ein DNA-Konstrukt, das Nukleotidsequenzen umfasst, die für eine Kombination von drei Proteinen für die Biosynthese von Apocarotinoidverbindungen, vorzugsweise Crocine, Crocetine, Picrocrocin, HTTC und Safranal, kodieren, wobei die Proteine aus UGT2, UGT709G1 und CCD2L oder einer funktionell äquivalenten Sequenz davon ausgewählt sind, und wobei diese Proteine vorzugsweise zur Gattung *Crocus,* vorzugsweise von *C. sativus,* gehören,
wobei die Aminosäuresequenzen für UGT2, UGT709G1 und CCD2L oder eine funktionell äquivalente Sequenz davon aus einer Aminosäuresequenz ausgewählt sind, die mindestens zu 90 %, 91 %, 92 %, 93 %, 94 %, 95 %, 96 %, 97 %, 98 % oder 99 % mit den Sequenzen identisch ist, die aus der Liste ausgewählt sind, bestehend jeweils aus SEQ ID NO: 4, SEQ ID NO: 6 und SEQ ID NO: 2,
wobei jede Nukleotidsequenz funktionsfähig mit einem Promotor verbunden ist, der ausgewählt ist aus einem fruchtspezifischen Promotor, einem knollenspezifischen Promotor und/oder einem konstitutiven Promotor, und
wobei die Nukleotidsequenz, die für CCD2L kodiert ist, funktionsfähig mit einem fruchtspezifischen Promotor oder einem knollenspezifischen Promotor verbunden ist.

2. Das DNA-Konstrukt gemäß Anspruch 1, wobei das UGT2-Protein durch eine Nukleotidsequenz kodiert ist, die mindestens 90%, 91%, 92%, 93%, 94%, 95%, 96%, 97%, 98% oder 99% Identität mit SEQ ID NO: 3 aufweist, das UGT709G1-Protein durch die Nukleotidsequenz kodiert ist, die mit mindestens 90%, 91%, 92%, 93%, 94%, 95%, 96%, 97%, 98% oder 99% Identität zu SEQ DI NO: 5 aufweist, und das CCD2L-Protein durch die Nukleotidsequenz kodiert ist, die mindestens 90%, 91%, 92%, 93%, 94%, 95%, 96%, 97%, 98% oder 99% Identität zu SEQ DI NO: 1 aufweist.

3. Das DNA-Konstrukt gemäß einem der Ansprüche 1 bis 2, wobei der fruchtspezifische Promotor aus der folgenden Liste ausgewählt ist: Tomaten-E8-Promotor (SEQ ID NO: 7) und p2A11-Promotor (SEQ ID NO: 8), der konstitutive Promotor der Cauliflower Mosaic Virus 35S-Promotor (SEQ ID NO: 9) ist und der knollenspezifische Promotor der Patatin-Promotor pPat (SEQ ID NO: 33) ist.

4. Das DNA-Konstrukt gemäß einem der Ansprüche 1 bis 3, wobei die Konstrukte aus der Liste ausgewählt sind bestehend aus SEQ ID NO: 21, SEQ ID NO: 22, SEQ ID
NO: 23, SEQ ID NO: 24 und SEQ ID NO: 34.

5. Ein Verfahren zur Erhöhung der Konzentrationen von Apocarotinoidverbindungen, vorzugsweise Crocinen, Picrocrocinen, HTTC und Safranal in einer Nachtschattenpflanze, wobei das Verfahren umfasst: Einbringen und Exprimieren des DNA-Konstrukts gemäß den Ansprüchen 1 bis 4 in die Nachtschattenpflanze.

6. Ein Verfahren zur Herstellung einer transgenen Nachtschattenpflanze mit erhöhten Gehalten an Apocarotinoidverbindungen, vorzugsweise Crocinen, Picrocrocinen, HTTC und Safranal, im Vergleich zur entsprechenden Wildtyp-Nachtschattenpflanze, wobei das Verfahren umfasst: Einführen und Exprimieren des DNA-Konstrukts gemäß den Ansprüchen 1-4 in die Nachtschattenpflanze.

7. Verfahren nach einem der Ansprüche 5 oder 6, wobei die Nachtschattenpflanze aus den Arten *Solanum tuberosum* (Kartoffelpflanze) und *Solanum lycopersicum* (Tomatenpflanze) ausgewählt ist.

8. Eine transgene Nachtschattenpflanze, die in ihrem Genom das DNA-Konstrukt gemäß einem der Ansprüche 1 bis 4 umfasst, wobei die Nachtschattenpflanze im Vergleich zur entsprechenden Wildtyp-Nachtschattenpflanze erhöhte Mengen an Apocarotinoidverbindungen, vorzugsweise Crocine, Picrocrocine, HTTC und Safranal, akkumuliert.

9. Die Transgene Nachtschattenpflanze gemäß Anspruch 8, wobei sie ausgewählt ist aus der Liste bestehend aus *Solanum tuberosum* (Kartoffelpflanze) und *Solanum lycopersicum* (Tomatenpflanze).

10. Die transgene Nachtschattenpflanze gemäß einem der Ansprüche 8 bis 9, wobei die erhöhten Gehalte an Apocarotinoidverbindungen, vorzugsweise Crocinen, Picrocrocinen, HTTC und Safranal, in den erntefähigen Teilen der Pflanze, vorzugsweise in Knollen, Fruchtgeweben oder Samen, erhöht sind.

11. Erntefähige Teile der Nachtschattenpflanze gemäß einem der Ansprüche 8 bis 10, ausgewählt aus Knollen, Samen und Früchten, mit erhöhten Gehalten an Apocarotinoidverbindungen.

12. Ein Lebensmittelprodukt, das den erntefähigen Teil der Nachtschattenpflanze gemäß Anspruch 11 umfasst, wobei das Lebensmittelprodukt einen erhöhten Gehalt an Apocarotinoidverbindungen, vorzugsweise Crocinen, Picrocrocinen, HTTC und Safranal, aufweist.

13. Das Lebensmittelprodukt gemäß Anspruch 12, wobei das Lebensmittelprodukt aus der Liste ausgewählt ist, die eine Tomate, ein Produkt auf Tomatenbasis, eine Kartoffel und ein Produkt auf Kartoffelbasis umfasst, wobei vorzugsweise das Produkt auf Tomatenbasis aus der Liste ausgewählt ist, die Ketchup, eine Sauce auf Tomatenbasis, eine Pizzasauce, Tomatensuppe oder Tomatensaft umfasst, und wobei vorzugsweise das Produkt auf Kartoffelbasis aus der Liste ausgewählt ist, die Kartoffelflocken, Kartoffelchips oder Kartoffelmehl umfasst.

14. Eine pharmazeutische oder nutrazeutische Zusammensetzung, die den erntefähigen Teil der Nachtschattenpflanze gemäß Anspruch 11 oder das Lebensmittelprodukt gemäß einem der Ansprüche 12 bis 13, ein Fragment oder einen Extrakt davon und einen pharmazeutisch oder nutrazeutisch akzeptablen Träger umfasst.

15. Der erntefähige Teil der Nachtschattenpflanze gemäß Anspruch 11, das Lebensmittel gemäß einem der Ansprüche 12 bis 13, die nutrazeutische Zusammensetzung oder die pharmazeutische Zusammensetzung gemäß Anspruch 14 zur Verwendung als Arzneimittel, vorzugsweise zur Verwendung bei der Vorbeugung und/oder Behandlung von neurodegenerativen Erkrankungen, vorzugsweise ausgewählt aus der Liste bestehend aus: Alzheimer-Krankheit und Parkinson-Krankheit.

## Revendications

1. Construction d'ADN comprenant des séquences de nucléotides codant pour une combinaison de trois protéines pour la biosynthèse de composés apocaroténoïdes, de préférence de crocines, crocétines, picrocrocine, HTTC et safranal, dans laquelle les protéines sont choisies parmi UGT2, UGT709G1 et CCD2L, ou une séquence fonctionnellement équivalente à celles-ci, et dans laquelle ces protéines de préférence appartiennent au genre *Crocus,* de préférence *C*. *sativus,*
dans laquelle les séquences d'acides aminés pour UGT2, UGT709G1 et CCD2L, ou une séquence fonctionnellement équivalente à celles-ci, sont choisies parmi une séquence d'acides aminés identique à au moins 90 %, 91 %, 92 %, 93 %, 94 %, 95 %, 96 %, 97 %, 98 % ou 99 % aux séquences choisies dans la liste constituée par les SEQ ID NO : 4, SEQ ID NO : 6 et SEQ ID NO : 2 respectivement,
dans laquelle chaque séquence de nucléotides est liée de manière fonctionnelle à un promoteur choisi parmi un promoteur spécifique d'un fruit, un promoteur spécifique d'un tubercule et/ou un promoteur constitutif, et
dans laquelle la séquence de nucléotides codant pour CCD2L est liée de manière fonctionnelle à un promoteur spécifique d'un fruit ou un promoteur spécifique d'un tubercule.

2. Construction d'ADN selon la revendication 1, dans laquelle la protéine UGT2 est codée par une séquence de nucléotides ayant une identité d'au moins 90 %, 91 %, 92 %, 93 %, 94 %, 95 %, 96 %, 97 %, 98 % ou 99 % avec la SEQ ID NO : 3, la protéine UGT709G1 est codée par la séquence de nucléotides ayant une identité d'au moins 90 %, 91 %, 92 %, 93 %, 94 %, 95 %, 96 %, 97 %, 98 % ou 99 % avec la SEQ ID NO : 5, et la protéine CCD2L est codée par la séquence de nucléotides ayant une identité d'au moins 90 %, 91 %, 92 %, 93 %, 94 %, 95 %, 96 %, 97 %, 98 % ou 99 % avec la SEQ ID NO : 1.

3. Construction d'ADN selon l'une quelconque des revendications 1 et 2, dans laquelle le promoteur spécifique d'un fruit est choisi dans la liste constituée par : le promoteur E8 (SEQ ID NO : 7) et le promoteur P2A11 (SEQ ID NO : 8) de la tomate, le promoteur constitutif est le promoteur 35S du virus de la mosaïque du chou-fleur (SEQ ID NO : 9) et le promoteur spécifique d'un tubercule est le promoteur de patatine pPat (SEQ ID NO : 33).

4. Construction d'ADN selon l'une quelconque des revendications 1 à 3, dans laquelle les constructions sont choisies dans la liste constituée par les SEQ ID NO : 21 ; SEQ ID NO : 22, SEQ ID NO : 23, SEQ ID NO : 24 et SEQ ID NO : 34.

5. Méthode pour augmenter les niveaux de composés apocaroténoïdes, de préférence de crocines, picrocrocines, HTTC et safranal, dans une plante solanacée, laquelle méthode comprend l'introduction et l'expression de la construction d'ADN selon les revendications 1 à 4 dans la plante solanacée.

6. Méthode pour produire une plante solanacée transgénique ayant des niveaux accrus de composés apocaroténoïdes, de préférence de crocines, picrocrocines, HTTC et safranal, en comparaison avec la plante solanacée de type sauvage correspondante, laquelle méthode comprend l'introduction et l'expression de la construction d'ADN selon les revendications 1 à 4 dans la plante solanacée.

7. Méthode selon l'une quelconque des revendications 5 et 6, dans laquelle la plante solanacée est choisie parmi les espèces *Solanum tuberosum* (plant de pomme de terre) et *Solanum lycopersicum* (plant de tomate).

8. Plante solanacée transgénique, qui comprend dans son génome la construction d'ADN selon l'une quelconque des revendications 1 à 4, laquelle plante solanacée accumule des niveaux accrus de composés apocaroténoïdes, de préférence de crocines, picrocrocines, HTTC et safranal, en comparaison avec la plante solanacée de type sauvage correspondante.

9. Plante solanacée transgénique selon la revendication 8, qui est choisie dans la liste constituée par *Solanum tuberosum* (plant de pomme de terre) et *Solanum lycopersicum* (plant de tomate).

10. Plante solanacée transgénique selon l'une quelconque des revendications 8 et 9, dans laquelle les niveaux accrus de composés apocaroténoïdes, de préférence de crocines, picrocrocines, HTTC et safranal, sont accrus dans des parties récoltables de la plante, de préférence dans les tubercules, les tissus de fruit, ou les graines.

11. Parties récoltables de la plante solanacée selon l'une quelconque des revendications 8 à 10, choisies parmi les tubercules, les graines et les fruits, ayant des niveaux accrus de composés apocaroténoïdes.

12. Produit alimentaire comprenant la partie récoltable des plantes solanacées selon la revendication 11, lequel produit alimentaire a des niveaux amplifiés de composés apocaroténoïdes, de préférence de crocines, picrocrocines, HTTC et safranal.

13. Produit alimentaire selon la revendication 12, lequel produit alimentaire est choisi dans la liste constituée par une tomate, un produit à base de tomate, une pomme de terre et un produit à base de pomme de terre, de préférence dans lequel le produit à base de tomate est choisi dans la liste constituée par le ketchup, une sauce à base de tomate, une sauce pour pizza, une soupe de tomate et un jus de tomate, et dans lequel de préférence le produit à base de pomme de terre est choisi dans la liste constituée par : un flocon de pomme de terre, une chips de pomme de terre, et une farine de pomme de terre.

14. Composition pharmaceutique ou nutraceutique comprenant la partie récoltable des plantes solanacées selon la revendication 11, ou le produit alimentaire selon l'une quelconque des revendications 12 et 13, un fragment ou un extrait de celui-ci, et un véhicule pharmaceutique ou nutraceutique acceptable.

15. Partie récoltable des plantes solanacées selon la revendication 11, produit alimentaire selon l'une quelconque des revendications 12 et 13, composition nutraceutique ou composition pharmaceutique selon la revendication 14, pour une utilisation en tant que médicament, de préférence pour une utilisation dans la prévention et/ou le traitement de maladies neurodégénératives de préférence choisies dans la liste constituée par : la maladie d'Alzheimer et la maladie de Parkinson.
